# EUROPEAN PATENT APPLICATION

(11) **EP 4 755 902 A1**
(43) Date of publication of application: **10.06.2026**
(21) Application number: 25807896.3
(22) Date of filing: 15.05.2025
(51) Int. Cl.: C07K 14/34, C12N 15/77, C12P 13/06, C07K 14/00

(54) **VARIANT OF PROTEIN AND METHOD FOR PRODUCING L-ISOLEUCINE BY USING SAME**

(30) Priority: 21.05.2024 KR 20240066104; 02.10.2024 KR 20240133999
(71) Applicant: CJ Cheiljedang Corporation, Seoul 04560 (KR)
(72) Inventor: YOON, Jeong-Hye, Seoul 04560 (KR); KIM, Bina, Seoul 04560 (KR); KANG, Hae-Won, Seoul 04560 (KR); CHOI, Jin-Geun, Seoul 04560 (KR)
(74) Representative: Meissner Bolte Partnerschaft mbB
(86) International application number: PCT/KR2025/006627
(87) International publication number: WO 2025/244356

(57) **Abstract**

The present disclosure relates to: a variant of a protein; a polynucleotide encoding the variant of a protein; a microorganism of the genus Corynebacterium, comprising at least one selected from the group consisting of the variant of a protein and the polynucleotide; and a method for producing L-isoleucine by using the microorganism.

## Description

### [TECHNICAL FIELD]

The present disclosure claims the benefit of the priority to Korean Patent Application No. 10-2024-0066104, filed on May 21, 2024, and Korean Patent Application No. 10-2024-0133999, filed on October 2, 2024, and the entire contents disclosed in the document of the corresponding Korean patent application are incorporated as a part of the present disclosure.

Throughout the present disclosure, numerous papers and patent documents are referenced and citations thereof are indicated. The disclosures of the cited papers and patent documents are incorporated into the present disclosure in their entirety by reference to more clearly describe the level of the technical field to which the present invention pertains and the contents of the present invention.

The present disclosure relates to a novel protein variant, a polynucleotide encoding the protein variant, a microorganism of the genus *Corynebacterium* comprising at least one selected from the group consisting of the protein variant and the polynucleotide encoding the protein variant, and a method of producing L-isoleucine using the microorganism.

### [BACKGROUND ART]

L-isoleucine (Ile, I) is classified as an essential amino acid, a type of branched-chain amino acid among the total twenty essential amino acids, and is used in the manufacture of various products such as animal feed, food additives, and pharmaceuticals. L-isoleucine has been increasingly used in the fields of intravenous solutions, nutritional supplements, sports nutritional supplements, as well as animal feed, because it functions in energy generation after metabolism, hemoglobin production, blood glucose regulation, generation and repair of muscle, and the like.

Based on this trend, various attempts have been made to improve production ability in methods for producing L-isoleucine using various microorganisms including microorganisms of the genus *Corynebacterium* (US6072083A).

Despite such efforts, the development of technology to improve L-isoleucine production ability is still required.

### [DISCLOSURE]

### [TECHNICAL PROBLEM]

One object of the present disclosure is to provide a novel protein variant.

Another object of the present disclosure is to provide a polypeptide encoding the protein variant.

Other object of the present disclosure is to provide a microorganism of the genus *Corynebacterium,* comprising at least one selected from the group consisting of the protein variant and the polynucleotides encoding the same.

Other object of the present disclosure is to provide a composition for producing L-isoleucine, comprising the microorganism of the genus *Corynebacterium.*

Other object of the present disclosure is to provide a method of producing L-isoleucine, comprising culturing the microorganism in a medium.

Other object of the present disclosure is to provide a use for producing L-isoleucine of the microorganism.

### [TECHNICAL SOLUTION]

Description thereof in detail is as follows. On the other hand, each description, aspect and embodiment disclosed in the present disclosure can also be applied to each other description, aspect and embodiment. In other words, all combinations of various elements disclosed in the present disclosure belong to the scope of the present disclosure. In addition, the scope of the present disclosure is not to be considered limited by the specific description described below. Furthermore, those skilled in the art can recognize or confirm numerous equivalents to the specific aspects of the present disclosure disclosed in the present disclosure using only common experiments. In addition, these equivalents are intended to be included in the present disclosure.

Furthermore, throughout the present disclosure, numerous papers and patent documents are referenced and citations thereof are indicated. The disclosures of the cited papers and patent documents are incorporated into the present disclosure in their entirety by reference to more clearly describe the level of the technical field to which the present disclosure pertains and the contents of the present disclosure.

One aspect of the present disclosure provides a protein variant comprising an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52.

In the present disclosure, the sequence of SEQ ID NO: 52 is shown in Table 1 below.

**[Table 1]**

| Protein name (gene ID, gene name) | Sequence (N-terminus → C-terminus) | SEQ ID NO: |
|---|---|---|
| Transporter (NCgl2828) | MMNSMSQAIDSKVEAHEGHEGHEGIERGTRNYKRA VFAMLAAGLAAFNGLYCTQALLPTMTEELGITPTESA LTVSATTGMLALCIVPASILSEKFGRGRVLTISLTLAIIV GLILPLVPNITALILLRGLQGALLAGTPAVAMTWLSEEI HPKDIGHAMGIYIAGNTVGGLTGRMIPAGLLEVTHW QNALLGSSIAALIFGVIMVVLLPKQRKFQPKNINLRHE | 52 |
| | ISAMAAHWRNPRLALLFGTAFLGMGTFVSLYNYLGF RMIDQFGLSEVLVGAVFIMYLAGTWSSTQAGALREKI GNGSTVIFLSLTMIASMALMGINNLWVTLVALFVFTA AFFALHSSASGWIGIIATKDRAEASSMYLFCYYVGSS VIGWVSGFAFTHLPWLAFIGWLILLLCGVLAICVTLAR LARNAN | |

In one embodiment, the afore-mentioned protein variant of the present disclosure may essentially consist of an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52. In another embodiment, the afore-mentioned protein variant of the present disclosure may consist of an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52.

The histidine (His, H), glutamic acid (Glu, E), and glycine (Gly, G) may be inserted sequentially in the order of histidine, glutamic acid, and glycine.

In the present disclosure, the amino acid sequence of SEQ ID NO: 52 may be the amino acid sequence of a transporter protein. In one embodiment, the protein may be a protein encoded by NCgl2828 gene.

In one embodiment, the amino acid corresponding to the 15th position from the N-terminus in the amino acid sequence of SEQ ID NO: 52 may be alanine (Ala, A), but not limited thereto.

In one embodiment, the amino acid corresponding to the 16th position from the N-terminus in the amino acid sequence of SEQ ID NO: 52 may be histidine (His, H), but not limited thereto.

The protein variant of the present disclosure may have an activity of increasing L-isoleucine production activity compared to a wild-type protein (polypeptide).

As used in the present disclosure, the term "variant" refers to a polypeptide in which one or more amino acids or nucleotides are conservatively substituted and/or modified such that the variant differs from the amino acid sequence or polynucleotide sequence prior to variation, while retaining the functions or properties thereof. Such variants may generally be identified by modifying one or more amino acids in the amino acid sequence of the polypeptide or one or more nucleotides in the nucleotide sequence of the polynucleotide, and evaluating the properties of the modified polypeptide or polynucleotide. That is, the ability of the variant may be increased, unchanged, or decreased relative to the polypeptide or polynucleotide prior to variation. In addition, some variants may include variants in which one or more portions, such as an N-terminal leader sequence or a transmembrane domain, or a nucleotide sequence encoding the leader sequence or transmembrane domain, are removed. Other variants may include variants in which a portion from the N- and/or C-terminus of a mature protein, or a nucleotide sequence encoding the same, is removed. The term "variant" may be used interchangeably with terms such as modification, modified polypeptide, modified protein, modified polynucleotide, mutant, mutein, divergent, and the like, and is not limited thereto as long as the term is used to indicate a modified or mutated form.

In addition, the variant may comprise deletion or addition of amino acids or nucleotides (in the case of a polynucleotide encoding the polypeptide) having minimal effects on the properties and secondary structure of the polypeptide. For example, a signal (or leader) sequence related to co-translational or post-translational translocation of proteins or a nucleotide sequence encoding the same may be conjugated at the N-terminus. Furthermore, the variant may be conjugated with another sequence or a linker to be identified, purified, or synthesized.

In one embodiment, the afore-mentioned protein variant of the present disclosure may have homology or identity of 70%, 75%, 80%, 85%, 90%, 91%, 92%, 93%, 94%, 95%, 96%, 97%, 98%, 99%, 99.1%, 99.2%, 99.25%, 99.275%, or 99.28% or more, and less than 100% to the amino acid sequence of SEQ ID NO: 52.

More specifically, the protein variant of the present disclosure may comprise an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52, and may have homology or identity of 90%, 91%, 92%, 93%, 94%, 95%, 96%, 97%, 98%, or 99%, at least 99.1%, 99.2%, 99.25%, 99.275%, or 99.28% or more, and less than 100%, to the amino acid sequence of SEQ ID NO: 52.

In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-isoleucine production) corresponding to the variant of the present disclosure.

For example, the variant may include sequence additions or deletions at the N-terminus, C-terminus, and/or within the amino acid sequence that do not alter the function of the variant of the present disclosure, as well as naturally occurring mutations, silent mutations, or conservative substitutions.

The "conservative substitution" refers to substituting one amino acid with an amino acid different from the original amino acid having similar structural and/or chemical characteristics. This amino acid substitution may occur generally based on the similarity in polarity, charge, solubility, hydrophobicity, hydrophilicity, and/or amphipathic nature of a residue. Commonly, the conservative substitution may hardly affect or not affect activity of a protein or polypeptide.

Another aspect of the present disclosure provides a protein variant, comprising at least one amino acid sequence selected from the group consisting of amino acid sequences of SEQ ID NOs: 1 to 5. In one embodiment, the protein variant may be a protein variant comprising the amino acid sequence of SEQ ID NO: 4. In other embodiment, the protein variant may essentially consist of the amino acid sequence of SEQ ID NO: 4. In other embodiment, the protein variant may consist of the amino acid sequence of SEQ ID NO: 4.

In the present disclosure, the amino acid sequence of SEQ ID NO: 1 may be a sequence of a protein variant in which the 118th amino acid of the amino acid sequence of protein encoded by NCgl0214 gene (membrane protein) is substituted from alanine (Ala, A) to valine (Val, V).

In the present disclosure, the amino acid sequence of SEQ ID NO: 2 may be a sequence of a protein variant in which the 38th amino acid of the amino acid sequence of protein encoded by NCgl0216 gene (hypothetical protein) is substituted from alanine (Ala, A) to threonine (Thr, T).

In the present disclosure, the amino acid sequence of SEQ ID NO: 3 may be a sequence of a protein variant in which the 303th amino acid of the amino acid sequence of protein encoded by NCgl2176 gene (ABC-type transporter, duplicated ATPase subunit) is substituted from valine (Val, V) to methionine (Met, M).

In the present disclosure, the amino acid sequence of SEQ ID NO: 4 may be a sequence of a protein variant in which histidine (His, H), glutamic acid (Glu, E), and glycine (Gly, G) are inserted between the 15th amino acid, alanine (Ala, A), and the 16th amino acid, histidine (His, H), of a protein (transporter protein) encoded by the NCgl2828 gene.

In the present disclosure, the amino acid sequence of SEQ ID NO: 5 may be a sequence of a protein variant of chorismate mutase encoded by the NCgl0819 gene (csm), resulting from substitution of the first nucleotide of the NCgl0819 gene (csm) from A to G.

The protein variant of the present disclosure may comprise any one amino acid sequence selected from the group consisting of amino acid sequences of SEQ ID NOs: 1 to 5 (for example, the amino acid sequence of SEQ ID NO: 4), or comprise an amino acid sequence having homology or identity of at least 70%, 75%, 80%, 85%, 90%, 95%, 96%, 97%, 98%, 99%, 99.5%, 99.7% or 99.9% or more to said amino acid sequence. In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-isoleucine production) corresponding to the variant of the present disclosure.

For example, the variant may include sequence additions or deletions at the N-terminus, C-terminus, and/or within the amino acid sequence that do not alter the function of the variant of the present disclosure, as well as naturally occurring mutations, silent mutations, or conservative substitutions.

Conservative substitutions, variants, and the like of the protein variant are as described above.

Another aspect of the present disclosure provides a polynucleotide variant comprising a nucleotide sequence selected from the group consisting of SEQ ID NOs: 11 to 14. The polynucleotide variant may encode rRNA. The rRNA encoded by the polynucleotide variant may exhibit improved L-isoleucine production ability (for example, increased production amount, increased production rate, increased production yield, or the like) compared to RNA prior to variation (for example, a corresponding wild-type RNA).

In one embodiment, the polynucleotide variant of the present disclosure may essentially consist of a nucleotide sequence selected from the group consisting of SEQ ID NOs: 11 to 14. In another embodiment, the polynucleotide variant of the present disclosure may consist of a nucleotide sequence selected from the group consisting of SEQ ID NOs: 11 to 14.

The polynucleotide variant of the present disclosure may comprise a nucleotide sequence selected from the group consisting of SEQ ID NOs: 11 to 14, or may comprise a nucleotide sequence having homology or identity of at least 70%, 75%, 80%, 85%, 90%, 95%, 96%, 97%, 98%, 99%, 99.5%, 99.7%, or 99.9% to the nucleotide sequence. In addition, it is apparent that any nucleotide sequence having such homology or identity and exhibiting the activity corresponding to the variant of the present disclosure (increased L-isoleucine production activity) is included within the scope of the present disclosure, even if part of the sequence is deleted, modified, substituted, conservatively substituted, or added.

For example, the variant may include sequence additions or deletions at the 5'-terminus, 3'-terminus, and/or within the nucleotide sequence that do not alter the function of the variant of the present disclosure, as well as naturally occurring mutations, silent mutations, or conservative substitutions.

Conservative substitutions, variants, and the like of the polynucleotide variant are as described above.

In the present disclosure, the term, 'homology' or 'identity' means the degree of similarity between two given amino acid sequences or nucleotide sequences, and may be expressed as a percentage. The terms, homology and identity may often be used interchangeably.

The sequence homology or identity of a conserved polynucleotide or polypeptide may be determined by a standard alignment algorithm, and a default gap penalty established by the program used may be used together. Substantially, a homologous or identical sequence may be generally hybridized with all or a part of a sequence under medium or high stringent conditions. It is obvious that hybridization also includes hybridization of a polynucleotide with a polynucleotide containing common codons or codons considering codon degeneracy.

Whether any two polynucleotide or polypeptide sequences have homology, similarity, or identity, may be determined using a known computer algorithm such as "FASTA" program using a default parameter such as that in for example, Pearson et al (1988) [Proc. Natl. Acad. Sci. USA 85]: 2444. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version) (GCG program package (including Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST or ClustalW of National Database Center of Biotechnology Information.

The homology, similarity, or identity of the polynucleotide or polypeptide may be determined by compared sequence information using for example, GAP computer program such as Needleman et al. (1970), J Mol Biol. 48:443, for example, known in Smith and Waterman, Adv. Appl. Math (1981) 2:482. In summary, the GAP program may be defined as a value of dividing a total number of symbols in the shorter of two sequences by the number of similar aligned symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

As one example of the present disclosure, the variant of the present disclosure may have activity to increase L-isoleucine production ability compared to a wild-type protein (polypeptide).

In the present disclosure, the term, "corresponding to" refers to an amino acid residue or a nucleotide at a position listed in a polypeptide or a polynucleotide, or an amino acid residue or a nucleotide similar or identical or homologous to a residue or a nucleotide listed in a polypeptide or a polynucleotide. Confirming an amino acid or a nucleotide at a corresponding position may determine a specific amino acid or a specificnucleotide of a sequence referring to a specific sequence. The "corresponding region" used in the present disclosure generally refers to a similar or corresponding position in a related protein, nucleic acid molecule, or reference protein or nucleic acid molecule.

For example, any amino acid sequence; or nucleotide sequence may be aligned with an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52, or any one amino acid sequence selected from the group consisting of SEQ ID NOs: 1 to 5 (for example, the amino acid sequence of SEQ ID NO: 4); or any one nucleotide sequence selected from the group consisting of SEQ ID NOs: 6 to 14, and based on this, each amino acid residue of the amino acid sequence or nucleotide may be numbered with reference to the number position of the amino acid residue or the nucleotide corresponding to an amino acid residue of an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52, or any one amino acid sequence selected from the group consisting of SEQ ID NOs: 1 to 5 (for example, the amino acid sequence of SEQ ID NO: 4); or any one nucleotide sequence selected from the group consisting of SEQ ID NOs: 6 to 14. For example, the sequence alignment algorithm as described in the present disclosure, may confirm a location of amino acids or nucleotides, or a location where modification such as substitution, insertion or deletion or the like occurs, compared to a query sequence (also called "reference sequence").

For this alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277) and the like may be used, but not limited thereto, and a sequence alignment program known in the art, pairwise sequence comparison algorithm and the like may be appropriately used.

Other aspect of the present disclosure provides a polynucleotide encoding the protein variant of the present disclosure.

In the present disclosure, the term, "polynucleotide" refers to a DNA or RNA strand of a certain length or more, which is a polymer of nucleotides in which nucleotide monomers are connected in a long chain form by covalent bonds, and more specifically, a polynucleotide fragment encoding the variant.

In one embodiment, the polynucleotide may be a polynucleotide encoding a protein variant comprising an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52.

In one embodiment, the polynucleotide may comprise a polynucleotide comprising any one nucleotide sequence selected from the group consisting of nucleotide sequences of SEQ ID NOs: 6 to 10, for example, the nucleotide sequence of SEQ ID NO: 9.

The polynucleotide encoding the protein variant of the present disclosure may have or comprise any one nucleotide sequence selected from the group consisting of nucleotide sequences of SEQ ID NOs: 6 to 10, for example, the nucleotide sequence of SEQ ID NO: 9. In another embodiment, the polynucleotide may comprise a nucleotide sequence having homology or identity of at least 70%, 75%, 80%, 85%, 90%, 95%, 96%, 97%, 98%, 99%, 99.5%, 99.7% or 99.9% or more to the nucleotide sequence. In addition, it is obvious that even a polynucleotide having a nucleotide sequence in which some sequences are deleted, modified, substituted, conservatively substituted, or added is included in the scope of the present disclosure, as long as it is a sequence which has such homology or identity and shows efficacy (efficacy of increasing L-isoleucine production) corresponding to the variant of the present disclosure.

In one embodiment, the nucleotide sequence or amino acid sequence provided in the present description may include one modified by common mutagenesis, for example, direct evolution and/or site-directed mutagenesis and the like, within a range of maintaining their original functions or desired functions. In one embodiment, that a polynucleotide or polypeptide "comprises a specific nucleotide or amino acid sequence" may mean that the polynucleotide or polypeptide (i) consists of the specific nucleotide sequence or amino acid sequence, or essentially comprises the same, or (ii) consists of an amino acid sequence having homology of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more to the specific nucleotide sequence or amino acid sequence or essentially comprises thereof and maintains the original functions and/or desired functions.

The polynucleotide of the present disclosure may have various modifications in coding regions within a range which does not change the amino acid sequence of the variant of the present disclosure, in consideration of degeneracy of codons or codons preferred in an organism in which the variant of the present disclosure is to be expressed. Specifically, the polynucleotide of the present disclosure may have or comprise a nucleotide sequence with homology or identity of 70%, 75%, 80%, 85%, 90%, 95%, 96%, 97%, or 98% or more, and less than 100% to any one nucleotide sequence selected from the group consisting of nucleotide sequences of SEQ ID NOs: 6 to 10 (for example, the nucleotide sequence of SEQ ID NO: 9), or consist of or essentially consist of a nucleotide sequence with homology or identity of 70%, 75%, 80%, 85%, 90%, 95%, 96%, 97%, or 98% or more, and less than 100% to the nucleotide sequence, but not limited thereto.

In addition, the polynucleotide of the present disclosure may comprise a probe that can be prepared from a known gene sequence, for example, any sequence capable of hybridizing with a complementary sequence to all or a part of the polynucleotide sequence of the present disclosure under stringent conditions without limitation. The "stringent condition" means a condition that allows specific hybridization between polynucleotides. For example, a condition of hybridizing polynucleotides with high homology or identity, polynucleotides with homology or identity of 70% or more, 75% or more, 80% or more, 85% or more, 90% or more, 95% or more, 96% or more, 97% or more, 98% or more, or 99% or more, and not hybridizing polynucleotides with homology or identity lower than that, or a condition of washing at a salt concentration and temperature corresponding to 60°C, 1×SSC, 0.1% SDS, specifically, 60°C, 0.1×SSC, 0.1% SDS, more specifically, 68°C, 0.1×SSC, 0.1% SDS, which is a washing condition of common southern hybridization, once, specifically, twice to 3 times may be listed.

Hybridization requires that two nucleotides have complementary sequences, but mismatches between bases may be allowed depending on the stringency of hybridization. The term, "complementary" may be used to describe relationship between nucleotide bases capable of hybridizing to each other. For example, in case of DNA, adenosine is complementary to thymine, and cytosine is complementary to guanine. Therefore, the polynucleotide of the present disclosure may also comprise not only a substantially similar nucleotide sequence, but also an isolated nucleic acid fragment complementary to the entire sequence.

Specifically, the polynucleotide with homology or identity to the polynucleotide of the present disclosure may be detected using a hybridization condition including a hybridization step at a Tm value of 55°C and using the afore-mentioned conditions. In addition, the value of Tm may be 60°C, 63°C or 65°C, but not limited thereto, and may be appropriately adjusted by those skilled in the art according to the purpose.

The suitable stringency to hybridize the polynucleotide depends on the length and degree of complementarity of the polynucleotide, and variables are well known in the art.

Other aspect of the present disclosure provides a vector comprising the polynucleotide or the polynucleotide variant of the present disclosure. The vector may be an expression vector to express the polynucleotide or the polynucleotide variant in a host cell, but not limited thereto.

The vector of the present disclosure may comprise a DNA product comprising a nucleotide sequence of a polynucleotide encoding the target polypeptide operably linked to an expression regulatory region (or expression regulatory sequence) suitable for expressing a target polypeptide in an appropriate host. The expression regulatory region may comprise a promoter that can initiate transcription, any operator sequence to regulate such transcription, a sequence encoding an appropriate mRNA ribosome binding site, and/or a nucleic acid sequence that regulates termination of transcription and translation. The vector may be transformed into a suitable host cell, and then be replicated or function independently of the host genome, and be integrated into the genome itself.

The vector used in the present disclosure is not particularly limited, and any vector known in the art may be used. Examples of the commonly used vector may include plasmids, cosmids, viruses, and bacteriophages in a natural state or a recombined state. For example, as the phage vector or cosmid vector, pWE15, M13, MBL3, MBL4, IXII, ASHII, APII, t10, t11, Charon4A, and Charon21A, and the like may be used, and as the plasmid vector, pDZ-based, pBR-based, pUC-based, pBluescriptll-based, pGEM-based, pTZ-based, pCL-based and pET-based and the like may be used. Specifically, pDZ, pDC, pDCM2, pACYC177, pACYC184, pCL, pECCG117, pUC19, pBR322, pMW118, pCC1BAC, or pDC24 vector, or the like may be used.

As one example, through a vector for inserting chromosome into a cell, a polynucleotide encoding a target polypeptide may be inserted into chromosome. Insertion of the polynucleotide into chromosome may be performed by any method known in the art, for example, homologous recombination, bout not limited thereto. A selection marker to confirm whether the chromosome is inserted may be further comprised. The selection marker is to select a cell transformed with a vector, that is, to confirm whether a target nucleic acid molecule is inserted, and markers which impart selectable phenotypes such as drug resistance, auxotrophy, resistance to a cytotoxic agent, or expression of a surface protein may be used. In an environment treated with a selective agent, only cells expressing the selection marker survive or show other phenotypes, so transformed cells may be selected.

In the present disclosure, the term, "transformation" means introducing a vector comprising a polynucleotide encoding a target polypeptide into a host cell or microorganism so that the polypeptide encoded by the polynucleotide is to be expressed in the host cell. As long as the transformed polynucleotide can be expressed in a host cell, all of them may be included regardless of whether they are inserted into chromosome of the host cell or located outside the chromosome. In addition, the polynucleotide comprises DNA and/or RNA encoding a target polypeptide. The polynucleotide may be introduced in any form, as long as it can be introduced into a host cell and expressed. For example, the polynucleotide may be introduced into a host cell in a form of expression cassette, which is a gene structure comprising all elements required for being expressed by itself. The expression cassette may commonly comprise a promoter operably linked to the polynucleotide, a transcription termination signal, a ribosome binding site and a translation termination signal. The expression cassette may be in a form of an expression vector capable of self-replication. In addition, the polynucleotide may be introduced into a host cell in its own form and be operably linked to a sequence required for expression in the host cell, but not limited thereto.

Furthermore, in the above, the term, "operably linked" refers to that the polynucleotide sequence is functionally linked to a promoter sequence which initiates and mediates transcription of the polynucleotide encoding the target variant of the present disclosure.

Other aspect of the present disclosure provides a microorganism of the genus *Corynebacterium,* comprising at least one selected from the group consisting of the protein variant of the present disclosure, the polynucleotide of the present disclosure, and the polynucleotide variant of the present disclosure.

The microorganism of the present disclosure may comprise a vector comprising the protein variant of the present disclosure, a polynucleotide encoding the protein variant, the polynucleotide of the present disclosure, or the polynucleotide variant of the present disclosure.

In the present disclosure, the term, "microorganism (or strain)" includes all of wild-type microorganisms or microorganisms in which genetic modification occurs naturally or artificially, and may be a microorganism of which specific mechanism is weakened or strengthened due to reasons such as insertion of a foreign gene or enhancement or inactivation of activity of an intrinsic gene and the like, which is a microorganism comprising genetic modification for production of a target polypeptide, protein or product.

The microorganism of the present disclosure may be a microorganism comprising any one or more of the protein variant of the present disclosure, the polynucleotide of the present disclosure, the polynucleotide variant of the present disclosure and a vector comprising the polynucleotide of the present disclosure or the polynucleotide variant of the present disclosure; a microorganism modified to express the protein variant of the present disclosure, the polynucleotide of the present disclosure or the polynucleotide variant of the present disclosure; a microorganism (for example, recombinant microorganism) expressing the variant of the present disclosure, the polynucleotide of the present disclosure, or the polynucleotide variant of the present disclosure; or a microorganism (for example, recombinant microorganism) having the activity of the protein variant or polynucleotide variant of the present disclosure, but not limited thereto.

The microorganism of the present disclosure may be a microorganism having L-isoleucine production ability.

The microorganism of the present disclosure may have increased **L-**isoleucine production ability compared to a microorganism of the genus *Corynebacterium* which does not comprise at least one selected from the group consisting of the protein variant of the present disclosure, a polynucleotide encoding the protein variant, and the polynucleotide variant of the present disclosure (i.e., a microorganism which does not express a protein variant comprising an amino acid sequence in which histidine (His, H), glutamic acid (Glu, E), and glycine (Gly, G) are inserted between the amino acids corresponding to the 15th and 16th positions from the N-terminus of the amino acid sequence of SEQ **ID** NO: 52; or an amino acid sequence selected from the group consisting of SEQ **ID** NOs: 1 to 5, for example the amino acid sequence of SEQ **ID** NO: 4; a microorganism which does not comprise nucleotide sequences of SEQ **ID** NOs: 6 to 14; a microorganism expressing a wild-type protein corresponding to the protein variant; or a microorganism expressing a wild-type polynucleotide corresponding to the polynucleotide variant).

The microorganism of the present disclosure may be a microorganism naturally having L-isoleucine production ability, or a microorganism into which the protein variant of the present disclosure, a polynucleotide encoding thereof, or the polynucleotide variant of the present disclosure (or a vector comprising the polynucleotide or the polynucleotide variant) has been introduced into a parent strain having no L-isoleucine production ability and/or to which L-isoleucine production ability is imparted, but not limited thereto.

As one example, the microorganism of the present disclosure is a cell or microorganism expressing the protein variant of the present disclosure, as transformed with a vector comprising the polynucleotide of the present disclosure, a polynucleotide encoding the variant of the present disclosure or the polynucleotide variant of the present disclosure, and for the purpose of the present disclosure, the microorganism of the present disclosure may include all microorganisms capable of producing L-isoleucine by comprising the variant of the present disclosure. For example, the microorganism of the present disclosure may be a recombinant microorganism having increased L-isoleucine production ability, obtained by introducing a polynucleotide encoding the protein variant of the present disclosure or the polynucleotide variant of the present disclosure into a natural wild-type microorganism or an L-isoleucine-producing microorganism.

In one specific embodiment, the microorganism of the present disclosure may have increased L-isoleucine production ability compared to a microorganism of the genus *Corynebacterium* which does not comprise at least one selected from the group consisting of the protein variant of the present disclosure, a polynucleotide encoding the protein variant, and the polynucleotide variant of the present disclosure. The microorganism of the genus *Corynebacterium* which does not comprise at least one selected from the group may be a natural wild-type microorganism or an unmodified microorganism, and may also be referred to as a parent strain.

In the present application, the microorganism as a host cell (unmodified microorganism) may be (1) a microorganism that naturally has L-isoleucine production ability, (2) a microorganism that naturally lacks or has significantly low L-isoleucine production ability, and/or (3) a microorganism in which a mutation is introduced (transformation) into a microorganism that naturally has L-isoleucine production ability or a microorganism that lacks or has significantly low L-isoleucine production ability, thereby acquiring or exhibiting enhanced L-isoleucine production ability. In one embodiment, the microorganism as a host cell may be *Corynebacterium glutamicum* KCCM12739P or *Corynebacterium glutamicum* KCCM11248P (KR 10-1335789 B), but is not limited thereto.

In one specific embodiment, the microorganism (a variant microorganism or a microorganism as a host cell) may be a microorganism in which the biosynthetic pathway of L-isoleucine is additionally enhanced to increase the production amount of L-isoleucine, but is not limited thereto.

In one specific embodiment, the microorganism may be a microorganism in which feedback inhibition of L-threonine dehydratase is released, and/or feedback inhibition of homoserine dehydrogenase is released.

In one specific embodiment, the microorganism may be a microorganism in which feedback inhibition of L-threonine dehydratase is released.

In the present disclosure, the term "threonine dehydratase (EC 4.3.1.19)" is an enzyme that produces 2-ketobutyrate from threonine, is encoded by the ilvA gene, and is known to be subjected to feedback inhibition by L-isoleucine. A microorganism of the genus *Corynebacterium* synthesizes L-isoleucine via three intermediate metabolites using pyruvate and 2-ketobutyrate as precursors. The amino acid sequence of the threonine dehydratase can be obtained from GenBank of NCBI, which is a known database, or from US 2023-0098971 A1, US 10982244 B2.

In another specific embodiment, the microorganism may be a microorganism in which feedback inhibition of homoserine dehydrogenase is released.

In the present disclosure, the term "homoserine dehydrogenase (EC:1.1.1.3)" is an enzyme which is encoded by the *hom* gene and catalyzes the synthesis of homoserine. Homoserine dehydrogenase is known to be subjected to feedback inhibition by isoleucine. The amino acid sequence of the homoserine dehydrogenase can be obtained from GenBank of NCBI, which is a known database, or from US 10982244 B2.

In one embodiment, the microorganism may be a microorganism in which feedback inhibition of aspartate kinase is released. In the present disclosure, the term "aspartate kinase (EC: 2.7.2.4)" is encoded by the lysC gene, and the amino acid sequence of the aspartate kinase can be obtained from GenBank of NCBI, which is a known database, or from US 10662450 B2.

In the present disclosure, the term, release of feedback inhibition, may mean that the activity of a polypeptide, protein, or enzyme is increased or enhanced compared to its intrinsic activity, or is no longer subject to inhibition relative to its intrinsic activity. In one embodiment, the microorganism may be a microorganism that further includes one or more mutations among the mutations of: additionally introducing a genetic mutation (R407H) into the *hom* gene (US 10982244 B2); additionally introducing a genetic mutation (T381A, F383A and/or V323A) into the *ilvA* gene (US 2023-0098971 A1, US 10982244 B2); and additionally introducing a genetic mutation (L377K) into the *lysC* gene encoding aspartate kinase (US 10662450 B2), but is not limited thereto.

In one embodiment, the microorganism of the present disclosure having increased L-isoleucine production amount may exhibit an increase in L-isoleucine production ability of about 1% or more, about 10% or more, about 20% or more, about 30% or more, about 40% or more, about 42.5% or more, about 42.75% or more, or about 42.8% or more compared to a parent strain prior to variation or an unmodified microorganism (the upper limit is not particularly limited and may be, for example, about 200% or less, about 150% or less, about 100% or less, or about 50% or less), but is not limited thereto. In another embodiment, the microorganism of the present disclosure having increased L-isoleucine production may exhibit an increase in L-isoleucine production ability of about 1.01-fold or more, about 1.1-fold or more, about 1.2-fold or more, about 1.3-fold or more, about 1.4-fold or more, about 1.425-fold or more, about 1.4275-fold or more, or about 1.428-fold or more compared to a parent strain prior to variation or an unmodified microorganism (the upper limit is not particularly limited and may be, for example, about 10-fold or less, about 5-fold or less, about 3-fold or less, or about 2-fold or less), but is not limited thereto.

The term "about" is a range including all of ±0.5, ±0.4, ±0.3, ±0.2, ±0.1, and the like, and includes all numerical values identical or similar to the numerical value appearing after the term of about, but is not limited thereto.

In one embodiment, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically, it may be *Corynebacterium glutamicum.*

In the present disclosure, the term, "weakening" of the polypeptide, is a concept including both reducing activity or having no activity compared to the intrinsic activity. The weakening may be interchangeably used with terms such as inactivation, deficiency, down-regulation, decrease, reduce, attenuation, and the like.

The weakening may also include cases where the activity of the polypeptide itself is reduced or removed compared to activity of the polypeptide possessed by the original microorganism due to mutation of a polynucleotide encoding the polypeptide and the like, cases where the overall polypeptide activity level and/or concentration (expression level) is lower than a natural strain in cells due to inhibition of expression or inhibition of translation into a polypeptide of a gene of a polynucleotide encoding the same, and the like, cases where expression of the polynucleotide is not done at all, and/or cases where there is no activity of the polypeptide even if the polynucleotide is expressed. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide is "inactivated, deficient, decreased, down-regulated, reduced, or attenuated" compared to the intrinsic activity, refers to that it is lowered compared to the activity of a specific polypeptide originally possessed by a parent strain before trait changes or non-modified microorganism.

This weakening of the activity of the polypeptide may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide may be
1) deficiency of the entire or a part of a gene encoding a polypeptide;
2) modification of an expression regulatory region (or expression regulatory sequence) so that expression of a gene encoding a polypeptide is reduced;
3) modification (for example, deletion/substitution/addition of at least one amino acid in the amino acid sequence) of an amino acid sequence composing the polypeptide so that the activity of the polypeptide is removed or weakened;
4) modification (for example, addition/substitution/addition of at least one nucleic acid base in the nucleic acid base sequence of the polypeptide gene to encode a polypeptide modified so that the activity of the polypeptide is removed or weakened) of a gene sequence encoding the polypeptide so that the activity of the polypeptide is removed or weakened;
5) modification of a base sequence encoding an initiation codon or 5'-UTR area of gene transcriptome encoding a polypeptide;
6) introduction of an antisense oligonucleotide (for example, antisense RNA) complementarily binding to transcriptome of a gene encoding a polypeptide;
7) addition of a sequence complementary to Shine-Dalgarno sequence in front of the Shine-Dalgarno sequence of a gene encoding a polypeptide so as to form a secondary structure incapable of attachment of ribosome;
8) addition of a promoter transcribed in the opposite direction at the 3' end of an ORF (open reading frame) of a gene sequence encoding a polypeptide (Reverse transcription engineering, RTE); or
9) adjustment of cellular localization of a protein (polypeptide); or
10) a combination of at least 2 selected from the 1) to 9), but not particularly limited thereto.

For example,
the 1) deficiency of the entire or a part of a gene encoding a polypeptide may be removal of the entire polynucleotide encoding an intrinsic target polypeptide in chromosome, replacement with a polynucleotide in which some nucleotides are deleted, or replacement with a marker gene.

In addition, the 2) modification of an expression regulatory region (or expression regulatory sequence) may be mutation occurrence on the expression regulatory region (or expression regulatory sequence) by deletion, insertion, non-conservative or conservative substitution or a combination thereof, or replacement with a sequence having weaker activity. The expression regulatory region includes a promoter, an operator sequence, a sequence encoding a ribosome binding site, and a sequence regulating termination of transcription and translation, but not limited thereto.

Furthermore, the 3) modification of a base sequence encoding an initiation codon or 5'-UTR area of gene transcriptome encoding a polypeptide may be for example, substituting it with a base sequence encoding another initiation codon with a lower polypeptide expression rate compared to the intrinsic initiation codon, but not limited thereto.

Moreover, the modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have weaker activity or an amino acid sequence or polynucleotide sequence improved to have no activity, but not limited thereto. For example, by forming a stop codon by introducing mutation in a polynucleotide sequence, expression of a gene may be inhibited or weakened, but not limited thereto.

The 6) introduction of an antisense oligonucleotide (for example, antisense RNA) complementarily binding to transcriptome of a gene encoding a polypeptide may refer to for example, the document [Weintraub, H. et al., Antisense-RNA as a molecular tool for genetic analysis, Reviews - Trends in Genetics, Vol. 1(1) 1986].

The 7) addition of a sequence complementary to Shine-Dalgarno sequence in front of the Shine-Dalgarno sequence of a gene encoding a polypeptide so as to form a secondary structure incapable of attachment of ribosome may make mRNA translation impossible or inhibit its speed.

The 8) addition of a promoter transcribed in the opposite direction at the 3' end of an ORF (open reading frame) of a gene sequence encoding a polypeptide (Reverse transcription engineering, RTE) may create an antisense nucleotide complementary to the transcriptome of the gene encoding the polypeptide to weaken the activity.

The 9) adjustment of cellular localization of a protein (polypeptide) may target a protein (polypeptide) to an intracellular specific organelle or specific intracellular space. For example, by addition or removal of a leader sequence functioning in targeting a protein (polypeptide), it may target it to periplasm or cytoplasm, but not limited thereto.

Such weakening of the activity of the polypeptide, may weaken the activity or concentration expression level of the corresponding polypeptide based on the activity or concentration of the expressed polypeptide in a wild-type or pre-transformed microbial strain, or decreasing the amount of products produced from the corresponding polypeptide, but is not limited thereto.

In the present disclosure, the term, "enhancement" of the polypeptide activity, means that the activity of the polypeptide is increased compared to the intrinsic activity. The enhancement may be interchangeably used with terms such as activation, up-regulation, overexpression, increase and the like. Herein, the activation, up-regulation, overexpression, and increase may include showing activity which is not originally present, or showing activity improved compared to the intrinsic activity or activity before modification. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the intrinsic activity, refers to that it is improved compared to the activity and/or concentration (expression level) of a specific polypeptide originally possessed by a parent strain before trait changes or non-modified microorganism.

The enhancement may be achieved by introducing a foreign polypeptide, or enhancement of activity of an intrinsic polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology, may be used, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be
1) an increase of a copy number in cells of a polynucleotide encoding a polypeptide;
2) replacement of a gene expression regulatory region on chromosome encoding a polypeptide with a sequence having strong activity;
3) modification of a base sequence encoding an initiation codon or 5'-UTR area of gene transcriptome encoding a polypeptide;
4) modification of an amino acid sequence of the polypeptide so that the activity of the polypeptide is enhanced;
5) modification of a polynucleotide sequence encoding the polypeptide so that the activity of the polypeptide is enhanced (for example, modification of a polynucleotide of the polypeptide gene so as to encode a polypeptide modified to enhance the activity of the polypeptide);
6) introduction of a foreign polypeptide showing activity of a polypeptide or a foreign polynucleotide encoding the same;
7) codon optimization of a polynucleotide encoding a polypeptide;
8) modification or chemical modification by analyzing the tertiary structure of a polypeptide and selecting an exposed site;
9) adjustment of cellular localization of a protein (polypeptide); or
10) a combination of at least 2 selected from the 1) to 9), but not particularly limited thereto.

The 1) increase of a copy number in cells of a polynucleotide encoding a polypeptide may be achieved by introduction of a vector which can replicate and function regardless of a host, in which a polynucleotide encoding the corresponding polypeptide is operably linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of gene transcriptome encoding a polypeptide may be for example, substituting it with a base sequence encoding another initiation codon with a higher polypeptide expression rate compared to the intrinsic initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an intrinsic polynucleotide is increased in a host cell, or codon optimization so that a foreign polynucleotide is optimized in a host cell to achieve transcription and translation.

The 8) modification or chemical modification by analyzing the tertiary structure of a polypeptide and selecting an exposed site may determine template protein candidates depending on the degree of similarity of sequences by comparing sequence information of a polypeptide to be analyzed, and confirm the structure based on this, and select an exposed site to be modified or chemically modified, and transform or modify it.

The 9) adjustment of cellular localization of a protein (polypeptide) may target a protein (polypeptide) into an intracellular specific organelle or specific intracellular space. For example, by addition or removal of a leader sequence functioning in targeting a protein (polypeptide), it may target it to periplasm or cytoplasm, but not limited thereto.

Such enhancement of the polypeptide activity means increasing activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the microorganism of the present disclosure, modification of a part or all of a polynucleotide (for example, modification to encode the afore-mentioned protein variant) may be induced by (a) homologous recombination using a vector for inserting chromosome into a microorganism or genome editing using genetic scissors (engineered nuclease, e.g., CRISPR-Cas9) and/or (b) treatment of light such as ultraviolet rays and radioactive rays and the like and/or chemical substances, but not limited thereto. The method of modifying a part of all of the gene may include a method for DNA recombination technology. For example, by injecting a nucleotide sequence or vector comprising a nucleotide sequence homologous to a target gene into the microorganism to cause homologous recombination, deletion of a part or all of the gene may be achieved. The injected nucleotide sequence or vector may include a dominant selection marker, but is not limited thereto.

In the microorganism of the present disclosure, the protein variant, polynucleotide and L-isoleucine and the like are as described in other aspects above.

Other aspect of the present disclosure provides a method for producing L-isoleucine, comprising culturing a microorganism of the genus *Corynebacterium* comprising the protein variant of the present disclosure, the polynucleotide of the present disclosure, or the polynucleotide variant in a medium.

The method for producing L-isoleucine of the present disclosure may comprise culturing a microorganism of the genus *Corynebacterium* comprising the protein variant of the present disclosure, the polynucleotide of the present disclosure, the polynucleotide variant of the present disclosure, or the vector of the present disclosure in a medium.

In the present disclosure, "culturing" means growing a microorganism of the genus *Corynebacterium* of the present disclosure under an appropriately adjusted environmental condition. The culturing process of the present disclosure may be conducted according to an appropriate medium and culturing conditions known in the art. This culturing process may be easily adjusted and used by those skilled in the art depending on the selected strain. Specifically, the culturing may be batch, continuous, and/or fed-batch, but not limited thereto.

In the present disclosure, the term "medium" refers to a material in which nutrients required for culturing the microorganism of the genus *Corynebacterium* are mixed as main components, and supplies nutrients and growth factors and the like including water which is essential for survival and growth. Specifically, the medium used for culturing the microorganism of the genus *Corynebacterium* of the present disclosure and other culturing conditions may be used as long as it is a medium used for culturing a common microorganism without particular limitation, but the microorganism of the genus *Corynebacterium* of the present disclosure may be cultured in a common medium containing a suitable carbon source, a nitrogen source, a phosphorus source, an inorganic compound, an amino acid and/or a vitamin and the like by adjusting temperature, pH and the like under an aerobic condition.

Specifically, the culture medium for the microorganism of the genus *Corynebacterium* may be found in the document ["Manual of Methods for General Bacteriology" by the American Society for Bacteriology (Washington D.C., USA, 1981)].

In the present disclosure, as the carbon source, carbohydrates such as glucose, saccharose, lactose, fructose, sucrose, maltose, etc.; sugar alcohols such as mannitol, sorbitol, etc., organic acids such as pyruvate, lactate, citrate, etc.; amino acids such as glutamic acid, methionine, lysine, etc. and the like, may be included. In addition, natural organic nutrients such as starch hydrates, molasses, blackstrap molasses, rice bran, cassava, sugar cane residues, and corn steep liquor may be used, and specifically, carbohydrates such as glucose and sterilized pre-treated molasses (that is, molasses converted with reducing sugars) and the like may be used, and other carbon sources in an appropriate amount may be variously used without limitation. These carbon sources may be used alone or at least two kinds may be used in combination, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; and organic nitrogen sources such as amino acids including glutamic acid, methionine, glutamine and the like, peptone, NZ-amine, meat extract, yeast extract, malt extract, corn steep liquor, casein hydrates, fish or degraded products thereof, defatted soybean cake or degraded products thereof, and the like may be used. These nitrogen sources may be used alone or at least two may be used in combination, but not limited thereto.

As the phosphorus source, potassium phosphate monobasic, potassium phosphate dibasic, or sodium-containing salts corresponding thereto, or the like may be included. As the inorganic compound, sodium chloride, calcium chloride, iron chloride, magnesium sulfate, iron sulfate, manganese sulfate, calcium carbonate and the like may be used, and in addition thereto, amino acids, vitamins, and/or appropriate precursors and the like may be included. These components or precursors may be added in a batch or continuous method. However, it is not limited thereto.

In addition, by adding a compound such as ammonium hydroxide, potassium hydroxide, ammonia, phosphoric acid, sulfuric acid and the like to a medium during culturing of the microorganism of the genus *Corynebacterium* of the present disclosure by an appropriate method, the pH of the medium may be adjusted. Furthermore, during culturing, foam generation may be inhibited using an antifoaming agent such as fatty acid polyglycol ester. Moreover, in order to maintain the aerobic state of the medium, oxygen or oxygen-containing gas may be injected into the medium, or in order to maintain the anaerobic and microaerobic state, gas may be not injected or nitrogen, hydrogen or carbon dioxide gas may be injected, but not limited thereto.

The culture temperature in the culturing of the present disclosure may be 20 to 45°C, specifically, at 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, but not limited thereto. In the culturing of the present disclosure, the time of culturing may be about 10 to 160 hours, but not limited thereto.

The L-amino acid produced by the culturing of the present disclosure may be released to a medium or remain in cells.

The method of producing L-amino acid of the present disclosure, may further comprise preparing the microorganism of the genus *Corynebacterium* of the present disclosure, preparing a medium for culturing the strain, or a combination thereof (in any order), for example, before the culturing.

The method of producing L-amino acid of the present disclosure, may further comprise recovering L-amino acid from the medium according to the culturing (medium in which culturing is performed) or the microorganism of the genus *Corynebacterium.* The recovering may be further comprised after the culturing.

The recovering may collect targeted L-amino acid using an appropriate method known in the art according to the culturing method of the microorganism of the present disclosure, for example, a batch, continuous or fed-batch culturing method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, targeted L-amino acids may be recovered from a medium or microorganism.

In addition, the method of producing L-amino acid of the present disclosure may additionally comprise a purification step. The purification may be performed, using an appropriate method known in the art. In one embodiment, when the method of producing L-amino acid of the present disclosure comprises both the recovering step and purification step, the recovering step and purification step may be continuously or non-continuously performed regardless of the order, or may be performed by being integrated as one step, but not limited thereto.

In the method of the present disclosure, the variant, polynucleotide, polynucleotide variant, vector and strain and the like are described in other aspects above.

Other aspect of the present disclosure provides a composition for producing L-isoleucine comprising a microorganism of the genus *Corynebacterium* which comprises at least one selected from the group consisting of the protein variant of the present disclosure, a polynucleotide encoding the protein variant, the polynucleotide variant of the present disclosure, a vector comprising the polynucleotide or the polynucleotide variant; a medium in which the microorganism is cultured; or a combination of two or more thereof.

The composition of the present disclosure may further comprise any appropriate excipient commonly used for compositions for producing amino acids, and such an excipient, may be for example, a preservative, wetting agent, dispersant, suspending agent, buffer, stabilizer, or tonicifying agent, or the like, but not limited thereto.

In the composition of the present disclosure, the variant, polynucleotide, vector, strain, medium and L-isoleucine and the like are as described in other aspects above.

In another aspect of the present disclosure, the present disclosure provides a method for increasing L-isoleucine production ability of a microorganism, a method for imparting L-isoleucine production ability to a microorganism, or a method for preparing a microorganism having increased L-isoleucine production ability, the method comprising introducing into the microorganism (for example, transforming the microorganism with) a novel protein variant of the present disclosure, a polynucleotide encoding the variant, a polynucleotide variant of the present disclosure, and/or a recombinant vector comprising the polynucleotide or the polynucleotide variant.

In the method for preparing the microorganism of the present disclosure, details regarding the protein variant, the polynucleotide encoding the same, the polynucleotide variant, the recombinant vector, and the microorganism are as described above.

According to other aspect of the present disclosure, the present disclosure provides a use of at least one selected from the group consisting of the protein variant of the present disclosure; a polynucleotide encoding the protein variant; a recombinant vector comprising the polynucleotide; and a microorganism comprising the protein variant, the polynucleotide encoding the protein variant, and/or the recombinant vector, for producing L-isoleucine, for preparing a microorganism producing L-isoleucine, and/or for imparting and/or increasing L-isoleucine production ability of a microorganism.

With respect to the above use for L-isoleucine production, preparing an L-isoleucine-producing microorganism, and/or imparting and/or increasing L-isoleucine production ability of a microorganism, details regarding the protein variant, the polynucleotide, the recombinant vector, and the microorganism are as described above.

According to other aspect of the present disclosure, the present disclosure provides a composition, method, product, process, or use characterized by at least one element disclosed in the present disclosure.

### [ADVANTAGEOUS EFFECTS]

When culturing a microorganism of the genus *Corynebacterium,* comprising the present variant of the present disclosure, high yield L-isoleucine production is possible compared to a microorganism having a conventional unmodified polypeptide.

### [MODE FOR INVENTION]

Hereinafter, the present disclosure will be described in more detail with reference to the following Examples. However, the following Examples are provided only to illustrate the present disclosure and are not intended to limit the scope of the present disclosure. Meanwhile, technical matters not specifically described herein can be sufficiently understood and readily implemented by a person skilled in the art of the present disclosure or in a similar technical field.

Throughout the present description, "%" used to indicate the concentration of a specific substance, unless otherwise mentioned, is (weight/weight) % for solid/solid, (weight/volume) % for solid/liquid, and (volume/volume) % for liquid/liquid.

### Examples

### Example 1: Selection of mutant strain with increased isoleucine productivity by artificial mutation

### Example 1-1: Random mutagenesis through UV irradiation

To select a mutant strain with increased isoleucine production ability, the L-isoleucine-producing strain *Corynebacterium glutamicum* KCCM12739P was smeared on a nutrient medium comprising agar and cultured at 30°C for 16 hours. Colonies obtained in this way were irradiated with UV (Ultraviolet mutation) at a room temperature and random mutation was induced on the genome in the strain.

### <Nutrient medium (pH 7.2)>

Glucose 10g, meat extract 5g, polypeptone 10g, sodium chloride 2.5g, yeast extract 5g, agar 20g, urea 2g (on the basis of 1 liter of distilled water)

### Example 1-2: Selection of strain with improved L-isoleucine productivity

To select mutant strains having increased L-isoleucine production ability compared to the parent strain KCCM12739P, the KCCM12739P strain and randomly mutagenized mutant strains were cultured as follows.

Each strain was inoculated into a 96-deep well plate (Bioneer) containing 400 µL of seed medium and cultured in a plate shaking incubator (TAITEC) at 32 °C and 1200 rpm for about 48 hours. The L-isoleucine concentrations of approximately 3,000 cultured strains were individually measured by NIR (near-infrared spectroscopy), and the top five mutant strains showing improved L-isoleucine production relative to the parent strain KCCM12739P were selected.

### <Seed medium (pH 7.0)>

Glucose 20 g, peptone 10 g, yeast extract 5 g, urea 1.5 g, KH₂PO₄ 4 g, K₂HPO₄ 8g, MgSO₄·7H₂O 0.5 g, biotin 100 µg , thiamine HCl 1000 µg , calcium-pantothenate 2000 µg, nicotinamide 2000 µg (on the basis of 1 liter of distilled water)

To finally select strains with reproducibly increased L-isoleucine production ability from the five selected mutant strains, they were cultured and evaluated by the following method.

The parent strain and the aforementioned mutant strains were inoculated into a 250 mL corner-baffled flask containing 25 mL of an isoleucine production medium, and then cultured with shaking at 32°C for 60 hours at 200 rpm. After the culture was completed, the L-isoleucine concentration in the culture broth was analyzed using HPLC, and the L-isoleucine production concentrations of each mutant strain are shown in Table 2 below.

### <Production Medium (pH 7.2)>

Glucose 10%, yeast extract 0.2%, ammonium sulfate 1.6%, monopotassium phosphate 0.1%, magnesium sulfate heptahydrate 0.1%, iron sulfate heptahydrate 10 mg/L, manganese sulfate monohydrate 10 mg/L, biotin 200 µg/L (based on 1 L of distilled water)

**[Table 2]**

| Strain name | L-isoleucine (g/L) |
|---|---|
| KCCM12739P | 2.0 |
| KCCM12739P_mt1 | 2.8 |
| KCCM12739P_mt2 | 2.6 |
| KCCM12739P_mt3 | 2.7 |
| **KCCM12739P_mt4** | **3.6** |
| KCCM12739P_mt5 | 2.5 |

Among the five selected mutant strains, KCCM12739P_mt4 was finally selected as the strain with significantly improved L-isoleucine production ability.

### Example 2. Mutation identification using whole-genome sequencing (WGS)

Whole-genome sequencing (WGS) was performed on the KCCM12739P_mt4 strain selected in Example 1-2 to analyze its sequence, and by comparison with the parent strain KCCM12739P, nucleotide variations occurring in CDS regions or rRNA regions were identified. The respective substitution types are shown in Tables 3 to 5 below.

**[Table 3]**

| Variations in the CDS (coding sequence) region | | | | | | | |
|---|---|---|---|---|---|---|---|
| **Wild-type strain amino acid** | **Mutation position in protein** | **Mutant strain amino acid** | **gene ID** | **Gene Name** | **Expressed protein** | **Variant amino acid sequence (SEQ ID NO.)** | **Variant nucleic acid sequence (SEQ ID NO.)** |
| A | 118 | V | NCgl0214 | - | membrane protein | 1 | 6 |
| A | 38 | T | NCgl0216 | - | hypothetical protein | 2 | 7 |
| V | 303 | M | NCgl2176 | - | ABC-type transporter, duplicated ATPase | 3 | 8 |
| | | | | | subunit | | |
| HEG inserted between the amino acids at positions 15 and 16 (mutation resulting from insertion of "CACGAAGGC" between nucleotides at positions 45 and 46 of the gene sequence) | | | NCgl2828 | - | transporter | 4 | 9 |
| M(atg) | 1 | M(gtg) | NCgl0819 | csm | Chorismate mutase | 5 | 10 |
| (mutation resulting from substitution of the first nucleotide of the gene sequence with G) | | | | | | | |

**[Table 4]**

| Variations in the rRNA region | |
|---|---|
| **gene ID** | **Variant nucleic acid sequence (SEQ ID NO.)** |
| NCg1r05 | 11 |
| NCg1r09 | 12 |
| NCg1r16 | 13 |
| NCg1r19 | 14 |

**[Table 5]**

| SEQ ID NO: | Sequence (amino acid sequence: N-terminus→ C-terminus; nucleotide sequence: 5'→3') |
|---|---|
| 1 | MLGSLWRFAVRTAAGAVALWVVIKLIDGISLSFPTTPLYQDGQHDNLLTFLAVAAIIV VLNATVKPVLKLLGLPLTIITLGLFSLVINAVIMLLAEYVSDLIGFGLRIETFGAAFWGV IVLALVNWVLGPITGLLGAKKD |
| 2 | MLGYIDEVGETGAFVSRQDPRYKTSPAFGYAGFLIPTENARAFGSEFTKQKRIRFS TEIVNAPNPARWEVKGADLFRPDTPIVRPENLRIFGSLVERLQKLGGHLFYYVDEK PIGTPRQTKLDQVGREREAMRETLNRLATQANREDRNLMVLVDSINEKSRKERIA EMYSHIFSRSGEHPEMRSIVEPPMHVDSALSSNIQFADWIAAYVSRAIDRQLIEDS RYQWIADDISTFKVYGSFTLESKLHLLRNRPCEDFNTSDILKRNRPMFSFDAATAIG SRVGVARAMQIKAASDRANSR |
| 3 | MAFADIVRSVENRTNAATLNWSIKNGWKPEVTGFSGYGSGRRVRVLARVLMSNP ENLLVDAPSQSITQQAQRGWRQFFTIQVPNLPVTVTVGGKTVTSSTNDNGYVDLL VEDHNLDPGWHTIQIQAEGSTPAEARVLIVENTARIGLISDIDDTIMVTWLPRALLAA WNSWVLHTNTRKPVPGMNRFYEELLKDHPDAPVFYLSTGAWNTFETLQEFINKH ALPDGPMLLTDWGPTPTGLFRSGQEHKKVQLRNLFIEYPDMKWILVGDDGQHDP LIYGEAVEEHPNRIAGVAIRELSPGEHMLSHGTTASLSTITTNGGQGVPVVHGRDG YELLQRYETKPFA |
| 4 | MMNSMSQAIDSKVEAHEGHEGHEGHEGIERGTRNYKRAVFAMLAAGLAAFNGLY CTQALLPTMTEELGITPTESALTVSATTGMLALCIVPASILSEKFGRGRVLTISLTLAII VGLILPLVPNITALILLRGLQGALLAGTPAVAMTWLSEEIHPKDIGHAMGIYIAGNTV GGLTGRMIPAGLLEVTHWQNALLGSSIAALIFGVIMVVLLPKQRKFQPKNINLRHEI SAMAAHWRNPRLALLFGTAFLGMGTFVSLYNYLGFRMIDQFGLSEVLVGAVFIMY LAGTWSSTQAGALREKIGNGSTVIFLSLTMIASMALMGINNLWVTLVALFVFTAAFF ALHSSASGWIGIIATKDRAEASSMYLFCYYVGSSVIGWVSGFAFTHLPWLAFIGWLI LLLCGVLAICVTLARLARNAN |
| 5 | MTNAGDNFEIRMPSGTDDPLSDAEIQKYREEINRLDREILDAVKRRTKISQTIGKTR MSSGGTRLVHTREVAIINQFREEIGEEGPALAGILLRMGRGKLG |
| 6 | ATGCTGGGTAGTCTTTGGCGTTTTGCGGTGCGCACCGCAGCAGGCGCGGTGG CGTTGTGGGTGGTTATTAAGCTTATCGACGGCATCTCCCTGAGTTTTCCCACC ACACCTCTCTATCAGGACGGTCAGCACGACAATCTGCTGACATTCCTGGCGGT GGCAGCAATCATTGTCGTGTTGAATGCCACGGTGAAACCCGTCTTGAAGCTGC TTGGTTTGCCGTTGACAATCATCACCTTGGGTCTGTTCTCGCTGGTCATCAAC GCGGTAATCATGCTGCTGGCGGAGTATGTGTCAGATTTGATCGGTTTCGGTCT ACGCATTGAAACCTTCGGTGCGGCCTTCTGGGGTGTGATTGTGCTGGCGTTA GTGAACTGGGTTCTTGGCCCCATTACCGGCCTCCTCGGTGCAAAAAGGACTA A |
| 7 | TTGCTTCTGGGTTATATTGACGAAGTGGGTGAAACTGGGGCATTCGTTTCTCG ACAGGATCCTCGATACAAGACATCACCGGCCTTCGGGTATGCCGGCTTCTTGA TACCAACTGAAAACGCTCGGGCGTTTGGTTCTGAATTTACAAAACAAAAGCGA ATCCGTTTTTCCACGGAAATAGTTAATGCACCTAATCCTGCAAGGTGGGAAGT GAAAGGAGCCGACCTCTTTCGTCCGGATACTCCAATTGTCCGACCTGAAAACC TTAGAATTTTTGGTTCTCTGGTAGAACGTCTTCAAAAACTGGGTGGACATCTAT TTTATTACGTCGATGAAAAACCAATCGGGACTCCACGACAGACGAAACTAGAT CAAGTAGGACGTGAACGGGAAGCAATGCGTGAGACACTCAATCGTCTCGCAA CTCAGGCGAATCGCGAAGATAGAAATCTAATGGTTCTGGTCGACAGCATCAAC GAAAAGTCTAGGAAAGAGCGAATCGCTGAAATGTATTCTCATATCTTTTCTAGA |
| | TCGGGAGAGCATCCAGAAATGCGTTCAATCGTTGAACCTCCCATGCATGTTGA TAGTGCATTGTCGTCGAACATTCAGTTTGCGGACTGGATTGCTGCCTATGTTTC ACGTGCTATAGATAGGCAACTAATTGAAGACTCCAGATATCAATGGATAGCGG ACGATATAAGTACGTTTAAAGTTTATGGTTCATTCACTTTGGAGTCTAAACTGCA TCTCTTACGGAATCGTCCTTGTGAAGATTTCAATACGAGCGACATATTAAAGAG AAATCGACCTATGTTTAGTTTTGATGCAGCTACTGCGATTGGTTCTCGCGTGG GGGTTGCGCGGGCGATGCAAATCAAGGCGGCTTCTGACAGGGCAAACTCTCG TTAG |
| 8 | ATGGCATTTGCAGACATTGTGCGCAGCGTCGAAAACCGCACCAACGCAGCGA CCCTCAACTGGTCCATCAAAAATGGCTGGAAGCCCGAAGTCACCGGATTTTCC GGGTACGGCTCCGGGCGTCGAGTGCGCGTCCTTGCGCGCGTGCTCATGTCC AACCCCGAAAATTTGCTTGTCGACGCCCCCTCCCAATCAATTACCCAACAAGC ACAGCGCGGTTGGCGCCAGTTCTTCACCATCCAAGTGCCCAACCTGCCAGTA ACTGTCACCGTTGGTGGGAAAACAGTTACCTCATCCACCAACGACAACGGCTA CGTTGACCTCCTGGTGGAAGACCACAACCTTGACCCCGGCTGGCACACCATC CAGATCCAAGCCGAAGGTTCCACCCCCGCCGAAGCCCGCGTCCTCATCGTGG AAAACACCGCCCGAATCGGACTCATCTCCGACATCGACGACACCATCATGGTC ACCTGGCTTCCCCGAGCACTCCTCGCCGCATGGAACTCGTGGGTTTTGCACA CCAACACCCGCAAACCAGTCCCCGGAATGAACCGCTTCTACGAAGAACTCCTC AAAGACCACCCCGACGCACCCGTGTTCTACCTCTCCACCGGCGCATGGAACA CCTTTGAAACCCTCCAAGAGTTCATCAACAAACACGCACTCCCCGACGGCCCC ATGCTGCTCACCGACTGGGGACCAACCCCCACAGGACTATTCCGCTCAGGTC AAGAGCACAAGAAAGTCCAACTGCGCAACCTGTTTATCGAATACCCCGACATG AAATGGATCCTCGTCGGCGACGATGGCCAACACGATCCCCTCATCTACGGCG AAGCAGTCGAAGAACACCCCAACCGCATCGCAGGCGTTGCAATCCGTGAGCT CTCCCCCGGCGAACATATGCTCTCCCACGGAACAACTGCGTCACTGTCCACCA TCACGACCAACGGGGGCCAAGGAGTCCCAGTAGTTCACGGCCGCGATGGATA TGAGTTGCTGCAGCGCTACGAGACGAAGCCGTTCGCCTGA |
| 9 | ATGATGAACTCCATGAGCCAAGCAATAGATAGCAAGGTCGAGGCACACGAAG GCCACGAAGGCCACGAAGGCCACGAAGGCATCGAGCGAGGAACACGCAATTA CAAGCGCGCTGTGTTTGCGATGCTGGCCGCCGGTCTTGCTGCTTTCAATGGT CTTTATTGCACGCAGGCATTGCTTCCCACCATGACGGAAGAGTTGGGAATTAC GCCCACTGAGTCCGCGCTGACGGTGTCGGCTACGACTGGAATGTTGGCGCTG TGTATTGTTCCGGCGTCGATACTTTCGGAGAAATTTGGTCGCGGTCGGGTGCT GACAATTTCACTCACGTTGGCCATCATCGTGGGATTAATTTTGCCGCTTGTCCC CAATATTACTGCTCTCATCCTGCTCAGAGGTCTCCAAGGTGCGCTGCTTGCTG |
| | GCACTCCAGCGGTGGCGATGACCTGGTTGTCTGAGGAAATTCACCCCAAGGA TATTGGGCATGCGATGGGAATTTACATCGCGGAAATACTGTCGGCGGCTC ACTGGACGTATGATTCCGGCGGACTACTTGAAGTAACTCATTGGCAAAACGC ACTGCTGGGAAGTTCTATCGCTGCGCTGATCTTCGGCGTAATCATGGTGGTGT TGCTTCCCAAGCAGCGGAAATTCCAGCCGAAGAATATCAATCTGCGCCATGAG ATTTCGGCGATGGCTGCTCATTGGCGGAATCCTCGTTTGGCGTTGCTTTTTGG TACTGCGTTTTTGGGCATGGGTACTTTTGTGTCGCTGTACAACTATTTGGGTTT CCGCATGATTGATCAGTTTGGGCTGAGTGAAGTGCTGGTTGGTGCGGTGTTCA TCATGTATCTGGCCGGGACCTGGAGTTCCACCCAGGCGGGTGCGTTGAGGGA GAAGATCGGCAATGGGTCAACGGTTATTTTCTTGAGTCTGACGATGATCGCGT CGATGGCACTGATGGGGATTAATAATTTGTGGGTCACGTTGGTTGCCCTGTTT GTGTTTACCGCGGCATTTTTCGCACTGCATTCCAGTGCTTCGGATGGATCGG AATCATCGCAACGAAGGATCGCGCGGAAGCCTCCAGCATGTATTTGTTCTGTT ATTACGTGGGATCCTCGGTGATTGGTTGGGTTTCTGGATTCGCGTTTACGCAT TTGCCGTGGTTGGCGTTCATTGGCTGGTTGATTCTGCTTCTTTGCGGAGTGCT GGCGATTTGTGTGACGCTGGCAAGGCTTGCCCGCAACGCCAATTAA |
| 10 | GTGACTAATGCAGGTGACAACTTCGAGATCAGGATGCCTTCTGGCACGGATGA CCCATTGTCCGATGCGGAGATCCAAAAGTATCGCGAGGAGATCAACCGCTTG GACCGCGAAATCCTCGATGCGGTGAAACGCCGCACGAAGATTTCCCAAACCA TCGGAAAAACACGCATGAGCTCGGGCGGAACACGTCTCGTGCACACCCGAGA AGTAGCAATCATCAACCAATTCCGTGAAGAGATCGGCGAGGAAGGCCCTGCC CTCGCTGGAATTTTGCTGCGCATGGACGCGGAAAACTCGGATAA |
| 11 | TTGTTGTTTTGTAGGGCACACGGTGGATGCCTTGGCATATCAAGCCGATGAAG GACGTGAGAGGCTGCGTTATGCCTCGGGGAGTTGCCAACTAAGCGTTGATCC GAGGATGTCCGAATGGGGAAACCCAGCCGCAGTGATGTGTGGTTACCTGCCA GTGAATGTATAGCTGGTGTGGAGGTTTACACGGGGAAGTGAAACATCTCAGTA CCCGTAGGAGAAGAAAACAATTGTGATTCCGTTAGTAGTGGCGAGCGAACGTG GATGATGGCTAAAACTTATGTGTGTGATACCCGGCAGGGGTTGCATGTAGGTG GTTGTGGGGCAATGACGTTCATATTCTGCCGGATGTGGGCATGTGCTGCGTG GTTAGTGGAAGTGGTGTGGAAACACCTGCCGTAGAAGGTGAGAGTCCTGTAC ACGAAGATCATGGTGGTGTGTGTGGTTGTTGATACCCCGAGTAGCAGCGGGC TCGTGGAATCTGCTGTGAATTAGCCGGGACCACCCGGTAAGCCTGAATACTTG ATATGACCGATAGCGGATTAGTACCGTGAGGGAATGGTGAAAAGTACCCCGG GAGGGGAGTGAAATAGTACCTGAAACCGTGTGCTGTCAATCCGTCAGAGCATC CTTTGTGGTGTGATGGCGTGCCTTTTGAAGAATGAGCCTGCGAGTCAGCGGC ATGTCGCGAGGTTAACCCGTGTGGGGTAGCCGTAGGGAAACCGAATCCTAAC |
| | GAGGGTGATTTTAGTGGCATGTCTTGGACCCGAAGCGGAGTGATCTACCCATG GCCAGTGTGAAGCAGCTGTAAGAGGTTGTGGAGGCGCGAACCCACTTAGGTT GAAAACTGAGGGGATGAGTTGTGGGTAGGGGTGAAAGGCCAATCAAACTCCG TGATAGCTGGTTCTCCCCGAAATGCATTTAGGTGCAGCGTCGTGTGTTTCTTG CCGGAGGTAGAGCTACTGGATGGTTTAGCGGGACCAACATCTTAGCGACATC AGCCAAACTCCGAATGCCGGTAAGTTAGAGCACGGCAGTGAGACTGCGGGGG ATAAGCTTCGTAGTCGAGAGGGAAACAGCCCAGATCGCCGGCTAAGGCCCCT AAGGGTGTGCTAAGTGGAAAAGGAGGTGGGGTCGCGAAGACAGCCAGGAGG TTGGCTTAGAAGCAGCCATCCTTGAAAGAGTGCGTAATAGCTCACTGGTCGAG TGATTCCGCGCCGACAATGTAGTGGGGCTTAAGTACACCGCCGAAGCCGCGG CAATGATCTTTATAGGATTGTTGGGTAGGGGAGCGTCGTGCATGCGTTGAAGC TTTGGGGTGACCTTGGGTGGAGTGTGTGCGAGTGAGAATGCAGGCATGAGTA ACGAATGATGCGTGAGAAACGTATCCGCCGGATGACTAAGGGTTCCTGGGTC AAGTTAATCTTCCCAGGGTGAGTCGGGGCCTAAGGCGAGGCCGACAGGCGTA GTCGATGGATAACGGGTTGATATTCCCGTACCCGAGTATGAGCGACCATGGTG AATCAGTGATACTAACCACCCATAAGCACCCGCGAAAAGGCTTTGCTTTTTTGT GGTGTGTGGTTGCGTGGGACCTGATCTGGTAGTAGCTAAGTGATGGGGTGAC GCAGGGAGGTAGCTCAGCCACTTATTGGATTGTGGTGTAAGCGTGTGGCACG CAGTGTTGGTAAATCCGCACTGTTTTTGTGTGAGGCGTGATGCGGAGCCCGTA AAGGGTGAAGTGGGTGATCCTGTGCTGTCGAGAAAAGCCTCTAGCGATGTTG ATATTCGGCCCGTACCCTAAACCGACACAGGTAGTCAGGTAGAGAATACTAAG GCGTTCGGGTGAACTGTGGTTAAGGAACTCGGCAAAATGCCCCCGTAACTTC GGGAGAAGGGGGGCCACGGCGTGTGAACAACTTTTCGTTGGGAGCGTGTTGT GGTCGCAGAGAATAGAGGGAAGCGACTGTTTACTAAAAACACAGGTCCGTGC GAAGACGTTTAAGTTGATGTATACGGACTGACGCCTGCCCGGTGCTGGAAGG TTAAGAGGACCGGTTAGCCGTAAGGCGAAGCTGAGAATTTAAGCCCCAGTAAA CGGCGGTGGTAACTATAACCATCCTAAGGTAGCGAAATTCCTTGTCGGGTAAG TTCCGACCTGCACGAATGGCGTAACGACTTCCCTGCTGTCTCAACCACAGGCC CGGTGAAATTGCAGTACGAGTAAAGATGCTCGTTACGCGCGGCAGGACGAAA AGACCCCGGGACCTTCACTATAGCTTGGTATTGGTGTTTGATTCGGTTTGTGTA GGATAGGTGGGAGACTGTGATCATATGACGCTAGTTGTGTGTGAGTCGTTGGT GAAATACCACTCTGATCGGATTGGATGTCTTAACCTTGGCCCATGATCTGGGT TGGGGACAGTGCCTGGTGGGTAGTTTAACTGGGGCGGTTGCCTCCTAAAATG TAACGGAGGCGCCCAAAGGTTTCCTCAGCTTGGTTGGTAATCAGGTGGTGAGT GTAAGTGCACAAGGGAGCTTGACTGTGACACTGACAGGTGGAGCAGGGACGA AAGTCGGGACTAGTGATCCGGCACCTACTTGTGGTTGTGGTGTCGCTCAACG |
| | GATAAAAGGTACCCCGGGGATAACAGGCTGATCTTCCCCAAGAGTCCATATCG ACGGGATGGTTTGGCACCTCGATGTCGGCTCGTCGCATCCTGGGGCTGGAGT AGGTCCCAAGGGTTGGGCTGTTCGCCCATTAAAGCGGCACGCGAGCTGGGTT TAGAACGTCGTGAGACAGTTCGGTCTCTATCCGCCGCGCGTTGAAACTTGA AGGAAGGCTGTCCCTAGTACGAGAGGACCGGGACGGACGTACCTCTGGTGTG CCAGTTGTTCCGCCAGGAGCAGGGCTGGTTGGCTACGTACGGAGGGATAAC CGCTGAAAGCATCTAAGCGGGAAGCCTGTTTCGAGATGAGGTTTCTTTTGAGG TTCCCTAGAGATTATGGGGTTGATAGGCCAGATCTGGAAGCACTGTGAGGTGT GGAGGTGACTGGTACTAATTTACCGATAACAACAAACC |
| 12 | TTGTTGTTTTGTAGGGCACACGGTGGATGCCTTGGCATATCAAGCCGATGAAG GACGTGAGAGGCTGCGTTATGCCTCGGGGAGTTGCCAACTAAGCGTTGATCC GAGGATGTCCGAATGGGGAAACCCAGCCGCAGTGATGTGGTTACCTGCCA GTGAATGTATAGCTGGTGTGGAGGTTTACACGGGGAAGTGAAACATCTCAGTA CCCGTAGGAGAAGAAAACAATTGTGATTCCGTTAGTAGTGGCGAGCGAACGTG GATGATGGCTAAAACTTATGTGTGATACCCGGCAGGGGTTGCATGTAGGTG GTTGTGGGGCAATAACATCCATATTCTGCCGGATGTGGGCATGTGCTGCGTG GTTAGTGGAAGTGGTGTGGAAACACCTGCCGTAGAAGGTGAGAGTCCTGTAC ACGAAGATCATGGTGGTGTGTGTGGTTGTTGATACCCCGAGTAGCAGCGGGC TCGTGGAATCTGCTGTGAATTAGCCGGGACCACCCGGTAAGCCTGAATACTTG ATATGACCGATAGCGGATTAGTACCGTGAGGGAATGGTGAAAAGTACCCCGG GAGGGGAGTGAAATAGTACCTGAAACCGTGTGCTGTCAATCCGTCAGAGCATC CTTTGTGGTGTGATGGCGTGCCTTTTGAAGAATGAGCCTGCGAGTCAGCGGC ATGTCGCGAGGTTAACCCGTGTGGGGTAGCCGTAGGGAAACCGAATCCTAAC GAGGGTGTTTTAGTGGCATGTCTTGGACCCGAAGCGGAGTGATCTACCCATG GCCAGTGTGAAGCAGCTGTAAGAGGTTGTGGAGGCGCAACCCACTTAGGTT GAAAACTGAGGGGATGAGTTGTGGGTAGGGGTGAAAGGCCAATCAAACTCCG TGATAGCTGGTTCTCCCCGAAATGCATTTAGGTGCAGCGTCGTGTGTTTCTTG CCGGAGGTAGAGCTACTGGATGGTTTAGCGGGACCAACATCTTAGCGACATC AGCCAAACTCCGAATGCCGGTAAGTGAGAGCACGGCAGTGAGACTGCGGGG GATAAGCTTCGTAGTCGAGAGGGAAACAGCCCAGATCGCCGGCTAAGGCCCC TAAGGGTGTGCTAAGTGGAAAAGGAGGTGGGGTCGCGAAGACAGCCAGGAG GTTGGCTTAGAAGCAGCCATCCTTGAAAGAGTGCGTAATAGCTCACTGGTCGA GTGATTCCGCGCCGACAATGTAGTGGGGCTTAAGTACACCGCCGAAGCCGCG GCAATGATCTTTATAGGATTGTTGGGTAGGGGAGCGTCGTGCATGCGTTGAAG CTTTGGGGTGACCTTGGGTGGAGTGTGTGCGAGTGAGAATGCAGGCATGAGT AACGAATGATGCGTGAGAAACGTATCCGCCGGATGACTAAGGGTTCCTGGGT |
| | CAAGTTAATCTTCCCAGGGTGAGTCGGGGCCTAAGGCGAGGCCGACAGGCGT AGTCGATGGATAACGGGTTGATATTCCCGTACCCGAGTATGAGCGACCATGGT GAATCAGTGATACTAACCACCCATAAGCACCCGCGAAAAGGCTTTGCTTTTTTG TGGTGTGTGGTTGCGTGGGACCTGATCTGGTAGTAGCTAAGTGATGGGGTGA CGCAGGGAGGTAGCTCAGCCACTTATTGGATTGTGGTGTAAGCGTGTGGCAC GCAGTGTTGGTAAATCCGCACTGTTTTTGTGTGAGGCGTGATGCGGAGCCCGT AAAGGGTGAAGTGGGTGATCCTGTGCTGTCGAGAAAAGCCTCTAGCGATGTT GATATTCGGCCCGTACCCTAAACCGACACAGGTAGTCAGGTAGAGAATACTAA GGCGTTCGGGTGAACTGTGGTTAAGGAACTCGGCAAAATGCCCCCGTAACTT CGGGAGAAGGGGGGCCACGGCGTGTGAACAACTTTTCGTTGGGAGCGTGTTG TGGTCGCAGAGAATAGAGGGAAGCGACTGTTTACTAAAAACACAGGTCCGTGC GAAGACGTTTAAGTTGATGTATACGGACTGACGCCTGCCCGGTGCTGGAAGG TTAAGAGGACCGGTTAGCCGTAAGGCGAAGCTGAGAATTTAAGCCCCAGTAAA CGGCGGTGGTAACTATAACCATCCTAAGGTAGCGAAATTCCTTGTCGGGTAAG TTCCGACCTGCACGAATGGCGTAACGACTTCCCTGCTGTCTCAACCACAGGCC CGGTGAAATTGCAGTACGAGTAAAGATGCTCGTTACGCGCGGCAGGACGAAA AGACCCCGGGACCTTCACTATAGCTTGGTATTGGTGTTTGATTCGGTTTGTGTA GGATAGGTGGGAGACTTCGATCATATGACGCTAGTTGTGTGTGAGTCGTTGGT GAAATACCACTCTGATCGGATTGGATGTCTTAACCTTGGCCCATGATCTGGGT TGGGGACAGTGCCTGGTGGGTAGTTTAACTGGGGCGGTTGCCTCCTAAAATG TAACGGAGGCGCCCAAAGGTTTCCTCAGCTTGGTTGGTAATCAGGTGGTGAGT GTAAGTGCACAAGGGAGCTTGACTGTGACACTGACAGGTGGAGCAGGGACGA AAGTCGGGACTAGTGATCCGGCACCTACTTGTGGTTGTGGTGTCGCTCAACG GATAAAAGGTACCCCGGGGATAACAGGCTGATCTTCCCCAAGAGTCCATATCG ACGGGATGGTTTGGCACCTCGATGTCGGCTCGTCGCATCCTGGGGCTGGAGT AGGTCCCAAGGGTTGGGCTGTTCGCCCATTAAAGCGGCACGCGAGCTGGGTT TAGAACGTCGTGAGACAGTTCGGTCTCTATCCGCCGCGCGCGTTGAAACTTGA AGGAAGGCTGTCCCTAGTACGAGAGGACCGGGACGGACGTACCTCTGGTGTG CCAGTTGTTCCGCCAGGAGCAGGGCTGGTTGGCTACGTACGGAGGGATAAC CGCTGAAAGCATCTAAGCGGGAAGCCTGTTTCGAGATGAGGTTTCTTTTGAGG TTCCCTAGAGATTATGGGGTTGATAGGCCAGATCTGGAAGCACTGTGAGGTGT GGAGGTGACTGGTACTAATTTACCGATAACAACAAACC |
| 13 | TGTGGAGAGTTTGATCCTGGCTCAGGACGAACGCTGGCGGCGTGCTTAACAC ATGCAAGTCGAACGCTGAAACCGGAGCTTGCTTTGGTGGATGAGTGGCGAAC GGGTGAGTAACACGTGGGTGATCTGCCCTACACTTTGGGATAAGCCTGGGAA ACTGGGTCTAATACCGAATATTCACACCACCGTAGGGGTGGTGTGGAAAGCTT |
| | TATGCGGTGTGGGATGAGCCTGCGGCCTATCAGCTTGTTGGTGGGGTAATGG CCTACCAAGGCGTCGACGGGTAGCCGGCCTGAGAGGGTGTACGGCCACATT GGGACTGAGACACGGCCCAGACTCCTACGGGAGGCAGCAGTGGGGAATATT GCACAATGGGCGCAAGCCTGATGCAGCGACGCCGCGTGGGGGATGAAGGCC TTCGGGTTGTAAACTCCTTTCGCTAGGGACGAAGCCTTATGGTGACGGTACCT GGAGAAGAAGCACCGGCTAACTACGTGCCAGCAGCCGCGGTAATACGTAGGG TGCGAGCGTTGTCCGGAATTACTGGGCGTAAAGAGCTCGTAGGTGGTTTGTC GCGTCGTCTGTGAAATCCCGGGGCTTAACTTCGGGCGTGCAGGCGATACGGG CATAACTTGAGTGCTGTAGGGGAGACTGGAATTCCTGGTGTAGCGGTGAAATG CGCAGATATCAGGAGGAACACCAATGGCGAAGGCAGGTCTCTGGGCAGTAAC TGACGCTGAGGAGCGAAAGCATGGGTAGCGAACAGGATTAGATACCCTGGTA GTCCATGCCGTAAACGGTGGGCGCTAGGTGTAGGGGTCTTCCACGACTTCTG TGCCGCAGCTAACGCATTAAGCGCCCCGCCTGGGGAGTACGGCCGCAAGGC TAAAACTCAAAGGAATTGACGGGGGCCCGCACAAGCGGCGGAGCATGTGGAT TAATTCGATGCAACGCGAAGAACCTTACCTGGGCTTGACATGGACCGGATCGG CGTAGAGATACGTTTTCCCTTGTGGTCGGTTCACAGGTGGTGCATGGTTGTCG TCAGCTCGTGTCGTGAGATGTTGGGTTAAGTCCCGCAACGAGCGCAACCCTT GTCTTATGTTGCCAGCACATTATGGTGGGTACTCATGAGAGACTGCCGGGGTT AACTCGGAGGAAGGTGGGGATGACGTCAAATCACATGCCCCTTATGTCCAGG GCTTCACACATGCTACAATGGTCGGTACAGCGAGTTGCCACACCGTGAGGTG GAGCTAATCTCTTAAAGCCGGCCTCAGTTCGGATTGGGGTCTGCAACTCGACC CCATGAAGTCGGAGTCGCTAGTAATCGCAGATCAGCAACGCTGCGGTGAATA CGTTCCCGGGCCTTGTACACACCGCCCGTCACGTCATGAAAGTTGGTAACACC CGAAGCCAGTGGCCCAACCTTTTAGGGGGGAGCTGTCGAAGGTGGGATCGG CGATTGGGACGAAGTCGTAACAAGGTAGCCGTACCGGAAGGTGCGGCTGGAT CACCTCCTTTCT |
| 14 | TGTGGAGAGTTTGATCCTGGCTCAGGACGAACGCTGGCGGCGTGCTTAACAC ATGCAAGTCGAACGCTGAAACCGGAGCTTGCTTTGGTGGATGAGTGGCGAAC GGGTGAGTAACACGTGGGTGATCTGCCCTACACTTTGGGATAAGCCTGGGAA ACTGGGTCTAATACCGAATATTCACACCACCGTAGGGGTGGTGTGGAAAGCCT TGTGCGGTGTGGGATGAGCCTGCGGCCTATCAGCTTGTTGGTGGGGTAATGG CCTACCAAGGCGTCGACGGGTAGCCGGCCTGAGAGGGTGTACGGCCACATT GGGACTGAGACACGGCCCAGACTCCTACGGGAGGCAGCAGTGGGGAATATT GCACAATGGGCGCAAGCCTGATGCAGCGACGCCGCGTGGGGGATGAAGGCC TTCGGGTTGTAAACTCCTTTCGCTAGGGACGAAGCCTTATGGTGACGGTACCT GGAGAAGAAGCACCGGCTAACTACGTGCCAGCAGCCGCGGTAATACGTAGGG |
| | TGCGAGCGTTGTCCGGAATTACTGGGCGTAAAGAGCTCGTAGGTGGTTTGTC GCGTCGTCTGTGAAATCCCGGGGCTTAACTTCGGGCGTGCAGGCGATACGGG CATAACTTGAGTGCTGTAGGGGAGACTGGAATTCCTGGTGTAGCGGTGAAATG CGCAGATATCAGGAGGAACACCAATGGCGAAGGCAGGTCTCTGGGCAGTAAC TGACGCTGAGGAGCGAAAGCATGGGTAGCGAACAGGATTAGATACCCTGGTA GTCCATGCCGTAAACGGTGGGCGCTAGGTGTAGGGGTCTTCCACGACTTCTG TGCCGCAGCTAACGCATTAAGCGCCCCGCCTGGGGAGTACGGCCGCAAGGC TAAAACTCAAAGGAATTGACGGGGGCCCGCACAAGCGGCGGAGCATGTGGAT TAATTCGATGCAACGCGAAGAACCTTACCTGGGCTTGACATGGACCGGATCGG CGTAGAGATACGTTTTCCCTTGTGGTCGGTTCACAGGTGGTGCATGGTTGTCG TCAGCTCGTGTCGTGAGATGTTGGGTTAAGTCCCGCAACGAGCGCAACCCTT GTCTTATGTTGCCAGCACATTGTGGTGGGTACTCATGAGAGACTGCCGGGGTT AACTCGGAGGAAGGTGGGGATGACGTCAAATCATCATGCCCCTTATGTCCAG GGCTTCACACATGCTACAATGGTCGGTACAGCGAGTTGCCACACCGTGAGGT GGAGCTAATCTCTTAAAGCCGGCCTCAGTTCGGATTGGGGTCTGCAACTCGAC CCCATGAAGTCGGAGTCGCTAGTAATCGCAGATCAGCAACGCTGCGGTGAAT ACGTTCCCGGGCCTTGTACACACCGCCCGTCACGTCATGAAAGTTGGTAACAC CCGAAGCCAGTGGCCCAACCTTTTAGGGGGGAGCTGTCGAAGGTGGGATCG GCGATTGGGACGAAGTCGTAACAAGGTAGCCGTACCGGAAGGTGCGGCTGGA TCACCTCCTTTCT |

In the following Examples, the effects of each variant shown in Tables 3 to 5 on the L-isoleucine production ability of a microorganism of the genus *Corynebacterium* were evaluated to identify effective factors influencing L-isoleucine production ability.

### Example 3: Preparation of an L-isoleucine-producing strain into which a variant gene was introduced

### Example 3-1: Construction of a recombinant vector for introduction of the variant gene

In order to confirm the effects of variants identified in Example 2, vectors capable of introducing them on chromosome were constructed.

Specifically, vectors containing each target mutation were constructed in order to insert the respective gene variants of NCgl0214, NCgl0216, NCgl2176, NCgl2828, NCgl0819, NCg1r05, NCg1r09, NCg1r16, and NCg1r19 into KCCM 12739P.

Specifically, genomic DNA of the KCCM12739P_mt4 strain was extracted using a G-spin Total DNA extraction mini kit (Intron, Cat. No. 17045) according to the protocol provided with the kit, and PCR was performed using the genomic DNA as a template. Solg^{™} Pfu-X DNA polymerase was used as the DNA polymerase. The PCR conditions were as follows: initial denaturation at 95 °C for 4 minutes; 27 cycles of denaturation at 95 °C for 30 seconds, annealing at 60 °C for 30 seconds, and extension at 72 °C for 2 minutes; followed by a final extension at 72 °C for 5 minutes. PCR products were obtained using primer pairs of SEQ ID NOs: 16 and 17; 18 and 19; 20 and 21; 22 and 23; 24 and 25; 26 and 27; 28 and 29; 30 and 31; or 32 and 33, respectively. The primer sequences used in the experiment are shown in Table 7 below.

The obtained mutation-containing fragments and the pDC24 vector (SEQ ID NO: 15, WO2024-242409 A1, Table 6) treated with the restriction enzyme Smal were cloned using a Gibson assembly method (D. G. Gibson et al., Nature Methods, Vol. 6, No. 5, May 2009; NEBuilder HiFi DNA Assembly Master Mix) to obtain recombinant plasmids. The resulting vectors comprising the respective mutation-containing fragments were designated pDC24-NCgl0214*, pDC24-NCgl0216*, pDC24-NCgl2176*, pDC24-NCgl2828*, pDC24-NCgl0819*, pDC24-NCg1r05*, pDC24-NCg1r09*, pDC24-NCg1r16*, and pDC24-NCg1r19*.

**[Table 6]**

| Sequence of pDC24 (SEQ ID NO: 15) (5'→3') |
|---|
| GTTCGCTTGCTGTCCATAAAACCGCCCAGTCTAGCTATCGCCATGTAAGCCCA CTGCAAGCTACCTGCTTTCTCTTTGCGCTTGCGTTTTCCCTTGTCCAGATAGC CCAGTAGCTGACATTCATCCGGGGTCAGCACCGTTTCTGCGGACTGGCTTTC TACGTGTTCCGCTTCCTTTAGCAGCCCTTGCGCCCTGAGTGCTTGCGGCAGC GTGAAGCTAGCTTTTATCGCCATTCGCCATTCAGGCTGCGCAACTGTTGGGAA GGGCGATCGGTGCGGGCCTCTTCGCTATTACGCCAGCTGGCGAAAGGGGGA TGTGCTGCAAGGCGATTAAGTTGGGTAACGCCAGGGTTTTCCCAGTCACGAC GTTGTAAAACGACGGCCAGTGAATTCGAGCTCGGTACCCGGGGATCCTCTAG |
| AGTCGACCTGCAGGCATGCAAGCTTGGCGTAATCATGGTCATAGCTGTTTCCT GTGTGAAATTGTTATCCGCTCACAATTCCACACAACATACGAGCCGGAAGCAT AAAGTGTAAAGCCTGGGGTGCCTAATGAGTGAGCTAACTCACATTAATTGCGT TGCGCTCACTGCCCGCTTTCCAGTCGGGAAACCTGTCGTGCCAGCTGCATTA ATGAATCGGCCAACGCGCGGGGAGAGGCGGTTTGCGTATTGGGCGCTCTTC CGCTTCCTCGCTCACTGACTCGCTGCGCTCGGTCGTTCGGCTGCGGCGAGC GGTATCAGCTCACTCAAAGGCGGTAATACGGTTATCCACAGAATCAGGGGAT AACGCAGGAAAGAACATGTGAGCAAAAGGCCAGCAAAAGGCCAGGAACCGTA AAAAGGCCGCGTTGCTGGCGTTTTTCCATAGGCTCCGCCCCCCTGACGAGCA TCACAAAAATCGACGCTCAAGTCAGAGGTGGCGAAACCCGACAGGACTATAA AGATACCAGGCGTTTCCCCCTGGAAGCTCCCTCGTGCGCTCTCCTGTTCCGA CCCTGCCGCTTACCGGATACCTGTCCGCCTTTCTCCCTTCGGGAAGCGTGGC GCTTTCTCAATGCTCACGCTGTAGGTATCTCAGTTCGGTGTAGGTCGTTCGCT CCAAGCTGGGCTGTGTGCACGAACCCCCCGTTCAGCCCGACCGCTGCGCCT TATCCGGTAACTATCGTCTTGAGTCCAACCCGGTAAGACACGACTTATCGCCA CTGGCAGCAGCCACTGGTAACAGGATTAGCAGAGCGAGGTATGTAGGCGGT GCTACAGAGTTCTTGAAGTGGTGGCCTAACTACGGCTACACTAGAAGGACAG TATTTGGTATCTGCGCTCTGCTGAAGCCAGTTACCTTCGGAAAAAGAGTTGGT AGCTCTTGATCCGGCAAACAAACCACCGCTGGTAGCGGTGGTTTTTTTGTTTG CAAGCAGCAGATTACGCGCAGAAAAAAAGGATCTCAAGAAGATCCTTTGATCT TTTCTACGGGGTCTGACGCTCAGTGGAACGAAAACTCACGTTAAGGGATTTTG GTCATGAGATTATCAAAAAGGATCTTCACCTAGATCCTTTTGGGGTGGGCGAA GAACTCCAGCATGAGATCCCCGCGCTGGAGGATCATCCAGCCCTGATAGAAA CAGAAGCCACTGGAGCACCTCAAAAACACCATCATACACTAAATCAGTAAGTT GGCAGCATCACCCGACGCACTTTGCGCCGAATAAATACCTGTGACGGAAGAT CACTTCGCAGAATAAATAAATCCTGGTGTCCCTGTTGATACCGGGAAGCCCTG GGCCAACTTTTGGCGAAAATGAGACGTTGATCGGCACGTAAGAGGTTCCAAC TTTCACCATAATGAAATAAGATCACTACCGGGCGTATTTTTTGAGTTATCGAGA TTTTCAGGAGCTGATAGAAACAGAAGCCACTGGAGCACCTCAAAAACACCATC ATACACTAAATCAGTAAGTTGGCAGCATCACCCGACGCACTTTGCGCCGAATA AATACCTGTGACGGAAGATCACTTCGCAGAATAAATAAATCCTGGTGTCCCTG TTGATACCGGGAAGCCCTGGGCCAACTTTTGGCGAAAATGAGACGTTGATCG GCACGTAAGAGGTTCCAACTTTCACCATAATGAAATAAGATCACTACCGGGCG TATTTTTTGAGTTATCGAGATTTTCAGGAGCTCTTTGGCATCGTCTCTCGCCTG TCCCCTCAGTTCAGTAATTTCCTGCATTTGCCTGTTTCCAGTCGGTAGATATTC CACAAAACAGCAGGGAAGCAGCGCTTTTCCGCTGCATAACCCTGCTTCGGGG TCATTATAGCGATTTTTTCGGTATATCCATCCTTTTTCGCACGATATACAGGAT TTTGCCAAAGGGTTCGTGTAGACTTTCCTTGGTGTATCCAACGGCGTCAGCCG GGCAGGATAGGTGAAGTAGGCCCACCCGCGAGCGGGTGTTCCTTCTTCACTG TCCCTTATTCGCACCTGGCGGTGCTCAACGGGAATCCTGCTCTGCGAGGCTG GCCGGCTACCGCCGGCGTAACAGATGAGGGCAAGCGGATGGCTGATGAAAC CAAGCCAACCAGGAAGGGCAGCCCACCTATCAAGGTGTACTGCCTTCCAGAC GAACGAAGAGCGATTGAGGAAAAGGCGGCGGCGGCCGGCATGAGCCTGTCG GCCTACCTGCTGGCCGTCGGCCAGGGCTACAAAATCACGGGCGTCGTGGAC TATGAGCACGTCCGCGAGGGCGTCCCGGAAAACGATTCCGAAGCCCAACCTT TCATAGAAGGCGGCGGTGGAATCGAAATCTCGTGATGGCAGGTTGGGCGTC GCTTGGTCGGTCATTTCGAAAAAGGTTAGGAATACGGTTAGCCATTTGCCTGC |
| TTTTATATAGTTCATATGGGATTCACCTTTATGTTGATAAGAAATAAAAGAAAAT GCCAATAGGATATCGGCATTTTCTTTTGCGTTTTTATTTGTTAACTGTTAATTGT CCTTGTTCAAGGATGCTGTCTTTGACAACAGATGTTTTCTTGCCTTTGATGTTC AGCAGGAAGCTCGGCGCAAACGTTGATTGTTTGTCTGCGTAGAATCCTCTGTT TGTCATATAGCTTGTAATCACGACATTGTTTCCTTTCGCTTGAGGTACAGCGAA GTGTGAGTAAGTAAAGGTTACATCGTTAGGCGGATCAAGATCCATTTTTAACA CAAGGCCAGTTTTGTTCAGCGGCTTGTATGGGCCAGTTAAAGAATTAGAAACA TAACCAAGCATGTAAATATCGTTAGACGTAATGCCGTCAATCGTCATTTTTGAT CCGCGGGAGTCAGTGAACAGGTACCATTTGCCGTTCATTTTAAAGACGTTCGC GCGTTCAATTTCATCTGTTACTGTGTTAGATGCAATCAGCGGTTTCATCACTTT TTTCAGTGTGTAATCATCGTTTAGCTCAATCATACCGAGAGCGCCGTTTGCTA ACTCAGCCGTGCGTTTTTTATCGCTTTGCAGAAGTTTTTGACTTTCTTGACGGA AGAATGATGTGCTTTTGCCATAGTATGCTTTGTTAAATAAAGATTCTTCGCCTT GGTAGCCATCTTCAGTTCCAGTGTTTGCTTCAAATACTAAGTATTTGTGGCCTT TATCTTCTACGTAGTGAGGATCTCTCAGCGTATGGTTGTCGCCTGAGCTGTAG TTGCCTTCATCGATGAACTGCTGTACATTTTGATACGTTTTTCCGTCACCGTCA AAGATTGATTTATAATCCTCTACACCGTTGATGTTCAAAGAGCTGTCTGATGCT GATACGTTAACTTGTGCAGTTGTCAGTGTTTGTTTGCCGTAATGTTTACCGGA GAAATCAGTGTAGAATAAACGGATTTTTCCGTCAGATGTAAATGTGGCTGAAC CTGACCATTCTTGTGTTTGGTCTTTTAGGATAGAATCATTTGCATCGAATTTGT CGCTGTCTTTAAAGACGCGGCCAGCGTTTTTCCAGCTGTCAATAGAAGTTTCG CCGACTTTTTGATAGAACATGTAAATCGATGTGTCATCCGCATTTTTAGGATCT CCGGCTAATGCAAAGACGATGTGGTAGCCGTGATAGTTTGCGACAGTGCCGT CAGCGTTTTGTAATGGCCAGCTGTCCCAAACGTCCAGGCCTTTTGCAGAAGA GATATTTTTAATTGTGGACGAATCAAATTCAGAAACTTGATATTTTTCATTTTTT TGCTGTTCAGGGATTTGCAGCATATCATGGCGTGTAATATGGGAAATGCCGTA TGTTTCCTTATATGGCTTTTGGTTCGTTTCTTTCGCAAACGCTTGAGTTGCGCC TCCTGCCAGCAGTGCGGTAGTAAAGGTTAATACTGTTGCTTGTTTTGCAAACT TTTTGATGTTCATCGTTCATGTCTCCTTTTTTATGTACTGTGTTAGCGGTCTGC TTCTTCCAGCCCTCCTGTTTGAAGATGGCAAGTTAGTTACGCACAATAAAAAAA GACCTAAAATATGTAAGGGGTGACGCCAAAGTATACACTTTGCCCTTTACACA TTTTAGGTCTTGCCTGCTTTATCAGTAACAAACCCGCGCGATTTACTTTTCGAC CTCATTCTATTAGACTCTCGTTTGGATTGCAACTGGTCTATTTTCCTCTTTTGTT TGATAGAAAATCATAAAAGGATTTGCAGACTACGGGCCTAAAGAACTAAAAAA TCTATCTGTTTCTTTTCATTCTCTGTATTTTTTATAGTTTCTGTTGCATGGGCAT AAAGTTGCCTTTTTAATCACAATTCAGAAAATATCATAATATCTCATTTCACTAA ATAATAGTGAACGGCAGGTATATGTGATGGGTTAAAAAGGATCACCCCAGAGT CCCGCTCAGAAGAACTCGTCAAGAAGGCGATAGAAGGCGATGCGCTGCGAAT CGGGAGCGGCGATACCGTAAAGCACGAGGAAGCGGTCAGCCCATTCGCCGC CAAGCTCTTCAGCAATATCACGGGTAGCCAACGCTATGTCCTGATAGCGGTC CGCCACACCCAGCCGGCCACAGTCGATGAATCCAGAAAAGCGGCCATTTTCC ACCATGATATTCGGCAAGCAGGCATCGCCATGGGTCACGACGAGATCCTCGC CGTCGGGCATCCGCGCCTTGAGCCTGGCGAACAGTTCGGCTGGCGCGAGCC CCTGATGCTCTTCGTCCAGATCATCCTGATCGACAAGACCGGCTTCCATCCGA GTACGTGCTCGCTCGATGCGATGTTTCGCTTGGTGGTCGAATGGGCAGGTAG CCGGATCAAGCGTATGCAGCCGCCGCATTGCATCAGCCATGATGGATACTTT CTCGGCAGGAGCAAGGTGAGATGACAGGAGATCCTGCCCCGGCACTTCGCC |
| CAATAGCAGCCAGTCCCTTCCCGCTTCAGTGACAACGTCGAGACAGCTGCGC AAGGAACGCCCGTCGTGGCCAGCCACGATAGCCGCGCTGCCTCGTCTTGGA GTTCATTCAGGGCACCGGACAGGTCGGTCTTGACAAAAAGAACCGGGCGCCC CTGCGCTGACAGCCGGAACACGGCGGCATCAGAGCAGCCGATTGTCTGTTGT GCCCAGTCATAGCCGAATAGCCTCTCCACCCAAGCGGCCGGAGAACCTGCGT GCAATCCATCTTGTTCAATCATGCGAAACGATCCTCATCCTGTCTCTTGATCAG ATCTTGATCCCCTGCGCCATCAGATCCTTGGCGGCAAGAAAGCCATCCAGTTT ACTTTGCAGGGCTTCCCAACCTTACCAGAGGGCGCCCCAGCTGGCAATTCCG |

**[Table 7]**

| SEQ ID NO: | Name | Sequence (5'->3') |
|---|---|---|
| 16 | NCgl0214_F | tgaattcgagctcggtacccATGCCACGTTTTCCC |
| 17 | NCgl0214_R | gtcgactctagaggatccccGCTGGGTAGTCTTTG |
| 18 | NCgl0216_F | tgaattcgagctcggtacccTTAGGACAGGGACGC |
| 19 | NCgl0216_R | gtcgactctagaggatccccAGAACCATTAGATTT |
| 20 | NCgl2176_F | tgaattcgagctcggtacccGCTTCTACGAAGAAC |
| 21 | NCgl2176_R | gtcgactctagaggatccccATCGGTTTTAAGAGC |
| 22 | NCgl2828_F | tgaattcgagctcggtacccTACGTTGGGGTGTTC |
| 23 | NCgl2828_R | gtcgactctagaggatccccGCCGGAATCATACGT |
| 24 | NCgl0819_F | tgaattcgagctcggtacccGCCCTGGTCAGGGTAT |
| 25 | NCgl0819_R | gtcgactctagaggatccccCCACCCACATGCGCT |
| 26 | NCg1r05_F | tgaattcgagctcggtacccACCTGCCGTAGAAGG |
| 27 | NCg1r05_R | gtcgactctagaggatccccAGCCGGCGATCTGGG |
| 28 | NCg1r09_F | tgaattcgagctcggtacccAGGCGTGATGCGGAG |
| 29 | NCg1r09_R | gtcgactctagaggatccccGCCCCAGTTAAACTA |
| 30 | NCg1r16_F | tgaattcgagctcggtacccTAATAAAGCTCCTTA |
| 31 | NCg1r16_R | gtcgactctagaggatccccACTGTCACCTTTGTG |
| 32 | NCg1r19_F | tgaattcgagctcggtacccCTACGAGCTCTTTAC |
| 33 | NCg1r19_R | gtcgactctagaggatccccTTTTTTAGAATCATT |

### Example 3-2: Preparation of an L-isoleucine-producing strain into which a variant gene was introduced

The nine vectors constructed in Example 3-1 were transformed into the L-isoleucine-producing strain KCCM12739P by electroporation (Appl. Microbiol. Biotechnol. (1999) 52:541-545), and strains in which the vectors were inserted into the chromosome by homologous recombination were selected on kanamycin-containing medium. Thereafter, transformants in which secondary recombination had been completed were analyzed by PCR using primer pairs of SEQ ID NOs: 34 and 35; 36 and 37; 38 and 39; 40 and 41; 42 and 43; 44 and 45; 46 and 47; 48 and 49; or 50 and 51 to confirm introduction of the variant genes. PCR was performed in the same manner as in Example 3-1, and the primer pairs used for the confirmation are shown in Table 8 below.

**[Table 8]**

| SEQ ID NO: | Name | Sequence (5' → 3') |
|---|---|---|
| 34 | NCgl0214_SEQ_F | CGGAGAGTTCGGTAATTTTC |
| 35 | NCgl0214_SEQ_R | TGGCGGCTAAATTGGCCGAG |
| 36 | NCgl0216_SEQ_F | CTGACTACGGATCAGAAGGT |
| 37 | NCgl0216_SEQ_R | ACATGCATGGGAGGTTCAAC |
| 38 | NCgl2176_SEQ_F | GACTCATCTCCGACATCGAC |
| 39 | NCgl2176_SEQ_R | AGTTAGTGCAAAAATATGTT |
| 40 | NCgl2828_SEQ_F | CGCCCACCCTAAAGAGGTAC |
| 41 | NCgl2828_SEQ_R | TTCTTCGGCTGGAATTTCCG |
| 42 | NCgl0819_SEQ_F | ACCTCCATTCGGGATTCTGC |
| 43 | NCgl0819_SEQ_R | CGATCATCGTGAGCAAACGC |
| 44 | NCg1r05_SEQ_F | CTAAAACTTATGTGTGTGAT |
| 45 | NCg1r05_SEQ_R | GGAATCACTCGACCAGTGAG |
| 46 | NCg1r09_SEQ_F | GGACCTGATCTGGTAGTAGC |
| 47 | NCg1r09_SEQ_R | GTCCCTGCTCCACCTGTCAG |
| 48 | NCg1r16_SEQ_F | GTTTGCTGTGTTGTTGTCAC |
| 49 | NCg1r16_SEQ_R | TCTGCTTGTTCCCCGTCAAG |
| 50 | NCg1r19_SEQ_F | CTGCGCATTTCACCGCTACA |
| 51 | NCg1r19_SEQ_R | TTATCCATCACTGTAAACAA |

The recombinant strains were designated KCCM12739P△NCgl0214::NCgl0214*, KCCM12739P△NCgl0216::NCgl0216*, KCCM12739P△NCgl2176::NCgl2176*, KCCM12739P△NCgl2828::NCgl2828*, KCCM12739P△NCgl0819::NCgl0819*, KCCM12739P△NCg1r05::NCgl1r05*, KCCM12739PΔNCg1r09::NCgl1r09*, KCCM12739PΔNCg1r16::NCgl1r16*, and KCCM12739PΔNCg1r19::NCgl1r19*, respectively.

### Example 4. Evaluation of L-isoleucine production ability of strains into which variant genes were introduced

To evaluate the L-isoleucine production ability of the strains prepared in Example 3-2 at the flask level, the strains were cultured and analyzed as follows.

The parent strain and the above mutant strains were inoculated into 250 mL corner-baffled flasks containing 25 mL of L-isoleucine production medium and cultured at 32 °C with shaking at 200 rpm for 60 hours. After completion of the culture, the amount of L-isoleucine produced was measured using high-performance liquid chromatography (HPLC), and the results are shown in Table 9 below.

### <Production Medium (pH 7.2)>

Glucose 10%, yeast extract 0.2%, ammonium sulfate 1.6%, monopotassium phosphate 0.1%, magnesium sulfate heptahydrate 0.1%, iron sulfate heptahydrate 10 mg/L, manganese sulfate monohydrate 10 mg/L, biotin 200 µg/L (based on 1 L of distilled water)

**[Table 9]**

| Strain name | L-isoleucine (g/L) |
|---|---|
| KCCM12739P | 2.1 |
| KCCM12739PΔNCgl0214:: NCgl0214* | 2.3 |
| KCCM12739PΔNCgl0216:: NCgl0216* | 2.3 |
| KCCM12739PΔNCgl2176:: NCgl2176* | 2.2 |
| **KCCM12739PΔNCgl2828:: NCgl2828*** | **3.0** |
| KCCM12739PΔNCgl0819:: NCgl0819* | 2.1 |
| KCCM12739PΔNCg1r05:: NCgl1r05* | 2.1 |
| KCCM12739PΔNCg1r09:: NCgl1r09* | 2.2 |
| KCCM12739PΔNCg1r16:: NCgl1r16* | 2.2 |
| KCCM12739PΔNCg1r19:: NCgl1r19* | 2.3 |

As shown in Table 9, some mutant strains (KCCM12739P△NCgl0819::NCgl0819* and KCCM12739P△NCg1r05::NCgl1r05*) exhibited L-isoleucine production ability comparable to that of the parent strain, whereas all of the remaining mutant strains showed superior L-isoleucine production ability relative to the parent strain. In particular, KCCM12739P△NCgl2828::NCgl2828* exhibited a markedly increased L-isoleucine production ability compared to the parent strain KCCM12739P.

From these results, it was confirmed that introduction of a protein variant in which histidine (His, H), glutamic acid (Glu, E), and glycine (Gly, G) are inserted between the 15th and 16th amino acids of the transporter protein encoded by the NCgl2828 gene enables more efficient production of L-isoleucine.

From the above description, those skilled in the art will understand that the present application can be implemented in other specific forms without altering its technical concept or essential characteristics. In this regard, it should be understood that the embodiments described above are illustrative in all respects and not restrictive. The scope of this application should be interpreted to include all changes or modifications derived from the meaning and scope of the following claims and their equivalents, rather than the detailed description above.

## Claims

1. A protein variant comprising an amino acid sequence in which histidine (His, H), glutamic acid (Glu, E), and glycine (Gly, G) are inserted between the amino acid corresponding to the 15th position and the amino acid corresponding to the 16th position from the N-terminus of the amino acid sequence of SEQ ID NO: 52.

2. The protein variant according to claim 1, wherein the protein variant has amino acid sequence identity of 80% or more to less than 100% to the amino acid sequence of SEQ ID NO: 52.

3. The protein variant according to claim 1, wherein the protein variant comprises the amino acid sequence of SEQ ID NO: 4.

4. A polynucleotide encoding the protein variant according to claim 1.

5. A microorganism of the genus *Corynebacterium* comprising at least one selected from the group consisting of the protein variant according to claim 1 and the polynucleotide encoding the protein variant.

6. The microorganism according to claim 5, wherein the microorganism of the genus *Corynebacterium* is *Corynebacterium glutamicum.*

7. The microorganism according to claim 5, wherein the microorganism has increased L-isoleucine productivity, compared to a microorganism of the genus *Corynebacterium* which does not comprise any one selected from the group consisting of the protein variant and the polynucleotide encoding the protein variant.

8. A method for producing L-isoleucine, comprising culturing the microorganism according to claim 5 in a medium.

9. The method for producing L-isoleucine according to claim 8, further comprising recovering L-isoleucine from the cultured medium or the cultured microorganism.

10. Use of the microorganism according to any one of claims 5 to 7 for producing L-isoleucine.
